# EUROPEAN PATENT APPLICATION

(11) **EP 4 648 598 A1**
(43) Date of publication of application: **12.11.2025**
(21) Application number: 25193494.9
(22) Date of filing: 22.09.2020
(51) Int. Cl.: H10K 85/60, C09K 11/06

(54) **ORGANIC LIGHT EMITTING DIODE AND ORGANIC LIGHT EMITTING DEVICE HAVING THE SAME**

(30) Priority: 02.10.2019 KR 20190121953
(62) Divisional of application: 20853610.2
(71) Applicant: LG Display Co., Ltd., Seoul, 07336 (KR); DuPont Specialty Materials Korea Ltd., Cheonan-si, Chungcheongnam-do 31093 (KR)
(72) Inventor: KIM, Do Han, 10845 Paju-si Gyeonggi-do (KR); KANG, Hye Seung, 10845 Paju-si Gyeonggi-do (KR); PARK, Kyoung Jin, 18449 Hwaseong-si Gyeonggi-do (KR); KIM, Chi Sik, 18449 Hwaseong-si Gyeonggi-do (KR); KIM, Hyun, 18449 Hwaseong-si Gyeonggi-do (KR)
(74) Representative: Viering, Jentschura & Partner mbB Patent- und Rechtsanwälte

(57) **Abstract**

The present disclosure relates to an organic light emitting diode that comprises an organic compound in which a carbazole moiety fused with an aromatic ring and a hetero aromatic ring is linked to a triazine moiety substituted with aromatic and/or hetero aromatic groups via an aromatic linker, and at least one protium constituting the aromatic and/or hetero aromatic groups of the triazine moiety and the aromatic linker moiety is substituted with deuterium, and an organic light emitting device including the diode. The molecular conformation of the deuterium-substituted triazine moiety and the aromatic linker moiety is not deteriorated and dissolved, and therefore, the organic compound can implement luminous efficiency and luminous lifetime as an organic compound in which whole protiums are substituted with deuteriums.

## Description

### Technical Field

This application claims the priority benefit of Korean Patent Application No. 10-2019-0121953, filed in the Republic of Korea on October 2, 2019, which is incorporated herein by reference in its entirety.

The present disclosure relates to a light emitting diode, and more particularly, to an organic light emitting diode having improved luminous efficiency and luminous life time and an organic light emitting device including the organic light emitting diode.

### Background Art

An organic light emitting diode (OLED) among a flat display device used widely has come into the spotlight as a display device replacing rapidly a liquid crystal display device (LCD). The OLED can be formed as a thin organic film less than 2000 Å and can implement unidirectional or bidirectional images by electrode configurations. Also, the OLED can be formed even on a flexible transparent substrate such as a plastic substrate so that a flexible or a foldable display device can be realized with ease using the OLED. In addition, the OLED can be driven at a lower voltage of 10 V or less so that the OLED has relatively lower power consumption for driving, and the OLED has excellent high color purity compared to the LCD.

Since fluorescent material uses only singlet exciton energy in the luminous process, the related art fluorescent material shows lower luminous efficiency than phosphorescent material. Metal complex, representative phosphorescent material, has too short luminous life time for commercial use. Therefore, there is a need to develop a new compound or a device structure that can enhance luminous efficiency and luminous lifetime of the organic light emitting diode.

### Disclosure of Invention

### Technical Problem

Accordingly, embodiments of the present disclosure are directed to an organic light emitting diode (OLED) and an organic light emitting device including the OLED that substantially obviates one or more of the problems due to the limitations and disadvantages of the related art.

An object of the present disclosure is to provide an OLED having improved luminous efficiency and luminous life time and an organic light emitting device including the OLED.

Another object of the present disclosure is to provide an OLED that can be fabricated at an economic cost and has excellent durability against external stress such as heat, and an organic light emitting device including the OLED.

Additional features and aspects will be set forth in the description that follows, and in part will be apparent from the description, or may be learned by practice of the inventive concepts provided herein. Other features and aspects of the inventive concept may be realized and attained by the structure particularly pointed out in the written description, or derivable therefrom, and the claims hereof as well as the appended drawings.

### Solution to Problem

To achieve these and other aspects of the inventive concepts, as embodied and broadly described, the present disclosure provides an organic light emitting diode that comprises an emitting material layer to which an organic compound having a triazine moiety substituted with an aromatic group and/or a hetero aromatic group and a carbazole moiety fused with an aromatic ring and a hetero aromatic ring and linked to the triazine moiety via an aromatic or hetero aromatic linker, wherein at least one of nuclear atoms among the aromatic and/or hetero aromatic groups substituted to the triazine moiety and the aromatic or hetero aromatic linker is substituted with deuterium is introduced.

As an example, at least one of the nuclear atoms constituting the aromatic and/or hetero aromatic groups substituted to the triazine moiety and/or at least one of the nuclear atoms constituting the aromatic or hetero aromatic linker may be substituted with deuterium.

In this case, the nuclear atoms constituting the aromatic rings of the carbazole moiety fused with the aromatic ring and the hetero aromatic ring may not be substituted with deuterium.

As an example, the organic compound may be used as a first host in the emitting material layer, and in this case, the emitting material layer may comprise a first dopant which may be green dopant.

Alternatively, the emitting material layer may comprise a second host of other green host, and/or may comprise a third host of yellow and/or red host and a second dopant of yellow and/or red dopant.

The organic light emitting diode may comprise a single emitting unit, or may have tandem structure including plural emitting units.

In another aspect, the present disclosure provides an organic light emitting device such as an organic light emitting luminescent device and an organic light emitting display device that includes the organic light emitting diode disposed over a substrate.

It is to be understood that both the foregoing general description and the following detailed description are exemplary and explanatory and are intended to provide further explanation of the inventive concepts as claimed.

### Advantageous Effects of Invention

At least one protium linked to the specific position in the organic compound, which is introduced to the emitting material layer, is substituted with deuterium. The deuterium has excellent resistance against external stress such as heat. In particular, the organic light emitting diode including an organic compound in which deuterium is substituted for the specific moiety where thermal decomposition can easily occur can improve its luminous efficiency and luminous life time similar to an organic light emitting diode including an organic compound in which all protium atoms constituting the entire molecule are substituted with deuterium.

It is possible to implement sufficiently improved luminous efficiency and luminous life time by introducing the organic compound substituted with deuterium to only some moieties according to the present disclosure, without substituting deuterium for the entire molecule. The present disclosure has an advantage of economical utilization of expensive deuterium, and therefore, can significantly reduce the manufacturing cost of the organic light emitting diode and the organic light emitting device.

### Brief Description of Drawings

The accompanying drawings, which are included to provide a further understanding of the disclosure, are incorporated in and constitute a part of this application, illustrate embodiments of the disclosure and together with the description serve to explain principles of the disclosure.
FIG. 1 is a schematic circuit diagram illustrating an organic light emitting display device of the present disclosure.
FIG. 2 is a schematic cross-sectional view illustrating an organic light emitting display device in accordance with a first aspect of the present disclosure.
FIG. 3 is a schematic cross-sectional view illustrating an OLED having single emitting unit in accordance with a first aspect of the present disclosure.
FIG. 4 is a schematic cross-sectional view illustrating an OLED having single emitting unit in accordance with a second aspect of the present disclosure.
FIG. 5 is a schematic cross-sectional view illustrating an organic light emitting display device in accordance with a second aspect of the present disclosure.
FIG. 6 is a schematic cross-sectional view illustrating an OLED having two emitting units in accordance with a third aspect of the present disclosure.
FIG. 7 is a schematic cross-sectional view illustrating an OLED having two emitting units in accordance with a fourth aspect of the present disclosure.
FIG. 8 is a schematic cross-sectional view illustrating an OLED having three emitting units in accordance with a fifth aspect of the present disclosure.
FIG. 9 is a schematic cross-sectional view illustrating an OLED having three emitting units in accordance with a sixth aspect of the present disclosure.
FIG. 10 is a schematic cross-sectional view illustrating an organic light emitting display device in accordance with a third aspect of the present disclosure.
FIGS. 11 to 14 are graph illustrating measurement results of voltage-current density, luminance-current efficiency, wavelength-luminescence peak intensity and time-luminance life time for an OLED fabricated in accordance with Example of the present disclosure.
FIGS. 15 to 18 are graph illustrating measurement results of voltage-current density, luminance-current efficiency, wavelength-luminescence peak intensity and time-luminance life time for an OLED fabricated in accordance with another Example of the present disclosure.
FIGS. 19 to 22 are graph illustrating measurement results of voltage-current density, luminance-current efficiency, wavelength-luminescence peak intensity and time-luminance life time for an OLED fabricated in accordance with still another Example of the present disclosure.

### Mode for the Invention

Reference will now be made in detail to aspects of the disclosure, examples of which are illillustrated in the accompanying drawings.

An organic light emitting diode (OLED) including an organic compound substituted partially with deuterium has improved luminous efficiency and luminous life time in accordance with the present disclosure. The OLED of the present disclosure may be applied to an organic light emitting device such as an organic light emitting display device or an organic light emitting lamination device. An organic light emitting display device including the OLED will be explained.

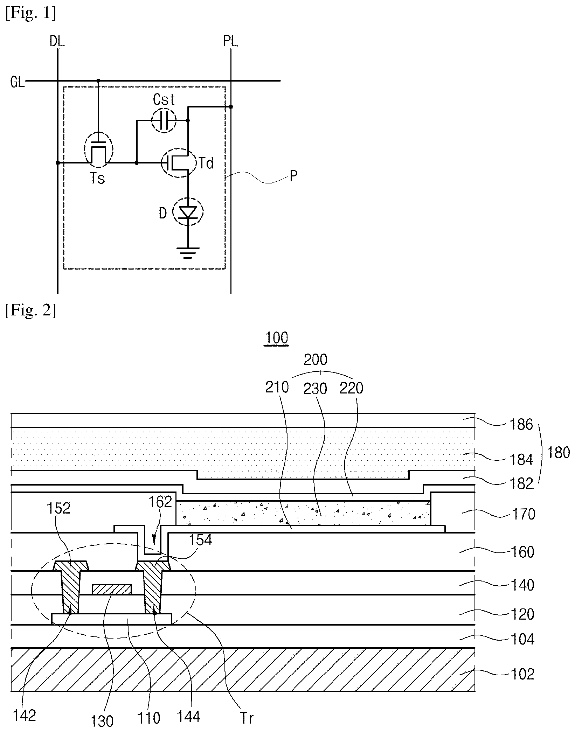

FIG. 1 is a schematic circuit diagram illustrating an organic light emitting display device of the present disclosure. As illustrated in FIG. 1, a gate line GL and a data line DL and power line PL, each of which cross each other to define a pixel region P, in the organic light display device. A switching thin film transistor Ts, a driving thin film transistor Td, a storage capacitor Cst and an OLED D are formed within the pixel region P. The pixel region P may include a red (R) pixel region, a green (G) pixel region and a blue (B) pixel region.

The switching thin film transistor Ts is connected to the gate line GL and the data line DL, and the driving thin film transistor Td and the storage capacitor Cst are connected between the switching thin film transistor Ts and the power line PL. The organic light emitting diode D is connected to the driving thin film transistor Td. When the switching thin film transistor Ts is turned on by a gate signal applied into the gate line GL, a data signal applied into the data line DL is applied into a gate electrode of the driving thin film transistor Td and one electrode of the storage capacitor Cst through the switching thin film transistor Ts.

The driving thin film transistor Td is turned on by the data signal applied into the gate electrode so that a current proportional to the data signal is supplied from the power line PL to the OLED D through the driving thin film transistor Td. And the organic light emitting diode D emits light having a luminance proportional to the current flowing through the driving thin film transistor Td. In this case, the storage capacitor Cst is charge with a voltage proportional to the data signal so that the voltage of the gate electrode in the driving thin film transistor Td is kept constant during one frame. Therefore, the organic light emitting display device can display a desired image.

FIG. 2 is a schematic cross-sectional view illustrating an organic light emitting display device in accordance with a first aspect of the present disclosure. As illustrated in FIG. 2, the organic light emitting display device 100 includes a substrate 102, a thin-film transistor Tr on the substrate 110, and an organic light emitting diode (OLED) 200 connected to the thin film transistor Tr.

The substrate 102 may include, but is not limited to, glass, thin flexible material and/ or polymer plastics. For example, the flexible material may be selected from the group, but is not limited to, polyimide (PI), polyethersulfone (PES), polyethylenenaphthalate (PEN), polyethylene terephthalate (PET), polycarbonate (PC) and combination thereof. The substrate 102, over which the thin film transistor Tr and the OLED 200 are arranged, form an array substrate.

A buffer layer 104 may be disposed over the substrate 102, and the thin film transistor Tr is disposed over the buffer layer 104. The buffer layer 104 may be omitted.

A semiconductor layer 110 is disposed over the buffer layer 104. In one exemplary aspect, the semiconductor layer 110 may include, but is not limited to, oxide semiconductor materials. In this case, a light-shield pattern may be disposed under the semiconductor layer 110, and the light-shield pattern can prevent light from being incident toward the semiconductor layer 110, and thereby, preventing the semiconductor layer 110 from being deteriorated by the light. Alternatively, the semiconductor layer 110 may include, but is not limited to, polycrystalline silicon. In this case, opposite edges of the semiconductor layer 110 may be doped with impurities.

A gate insulating layer 120 formed of an insulating material is disposed on the semiconductor layer 110. The gate insulating layer 120 may include, but is not limited to, an inorganic insulating material such as silicon oxide (SiO ₓ) or silicon nitride (SiN ₓ).

A gate electrode 130 made of a conductive material such as a metal is disposed over the gate insulating layer 120 so as to correspond to a center of the semiconductor layer 110. While the gate insulating layer 120 is disposed over a whole area of the substrate 102 in FIG. 1, the gate insulating layer 120 may be patterned identically as the gate electrode 130.

An interlayer insulating layer 140 formed of an insulating material is disposed on the gate electrode 130 with covering over an entire surface of the substrate 102. The interlayer insulating layer 140 may include, but is not limited to, an inorganic insulating material such as silicon oxide (SiO ₓ) or silicon nitride (SiN ₓ), or an organic insulating material such as benzocyclobutene resin or photo-acryl.

The interlayer insulating layer 140 has first and second semiconductor layer contact holes 142 and 144 that expose both sides of the semiconductor layer 110. The first and second semiconductor layer contact holes 142 and 144 are disposed over opposite sides of the gate electrode 130 with spacing apart from the gate electrode 130. The first and second semiconductor layer contact holes 142 and 144 are formed within the gate insulating layer 120 in FIG. 1. Alternatively, the first and second semiconductor layer contact holes 142 and 144 are formed only within the interlayer insulating layer 140 when the gate insulating layer 120 is patterned identically as the gate electrode 130.

A source electrode 152 and a drain electrode 154, which are formed of conductive material such as a metal, are disposed on the interlayer insulating layer 140. The source electrode 152 and the drain electrode 154 are spaced apart from each other with respect to the gate electrode 130, and contact both sides of the semiconductor layer 110 through the first and second semiconductor layer contact holes 142 and 144, respectively.

The semiconductor layer 110, the gate electrode 130, the source electrode 152 and the drain electrode 154 constitute the thin film transistor Tr, which acts as a driving element. The thin film transistor Tr in FIG. 1 has a coplanar structure in which the gate electrode 130, the source electrode 152 and the drain electrode 154 are disposed over the semiconductor layer 110. Alternatively, the thin film transistor Tr may have an inverted staggered structure in which a gate electrode is disposed under a semiconductor layer and a source and drain electrodes are disposed over the semiconductor layer. In this case, the semiconductor layer may comprise amorphous silicon.

A gate line GL and a data line DL, which cross each other to define a pixel region P, and a switching element Ts, which is connected to the gate line GL and the data line DL is, may be further formed in the pixel region P. The switching element Ts is connected to the thin film transistor Tr, which is a driving element. Besides, the power line PL is spaced apart in parallel from the gate line GL or the data line DL, and the thin film transistor Tr may further include a storage capacitor Cst configured to constantly keep a voltage of the gate electrode 130 for one frame.

A passivation layer 160 is disposed on the source and drain electrodes 152 and 154 over the whole substrate 102. The passivation layer 160 has a flat top surface and a drain contact hole 162 that exposes the drain electrode 154 of the thin film transistor Tr. While the drain contact hole 162 is disposed on the second semiconductor layer contact hole 144, it may be spaced apart from the second semiconductor layer contact hole 144.

The OLED 200 includes a first electrode 210 that is disposed on the passivation layer 160 and connected to the drain electrode 154 of the thin film transistor Tr. The OLED 200 further includes an organic emissive layer 230 and a second electrode 220 each of which is disposed sequentially on the first electrode 210.

The first electrode 210 is disposed in each pixel region. The first electrode 210 may be an anode and include a conductive material having a relatively high work function value. For example, the first electrode 210 may include, but is not limited to, a transparent conductive material (TCO) such as indium tin oxide (ITO), indium zinc oxide (IZO), indium tin zinc oxide (ITZO), tin oxide (SnO), zinc oxide (ZnO), indium cerium oxide (ICO), aluminum doped zinc oxide (AZO), and the like.

In one exemplary aspect, when the organic light emitting display device 100 is a top-emission type, a reflective electrode or a reflective layer may be disposed under the first electrode 210. For example, the reflective electrode or the reflective layer may include, but are not limited to, aluminum-palladium-copper (APC) alloy.

In addition, a bank layer 170 is disposed on the passivation layer 160 in order to cover edges of the first electrode 210. The bank layer 170 exposes a center of the first electrode 210.

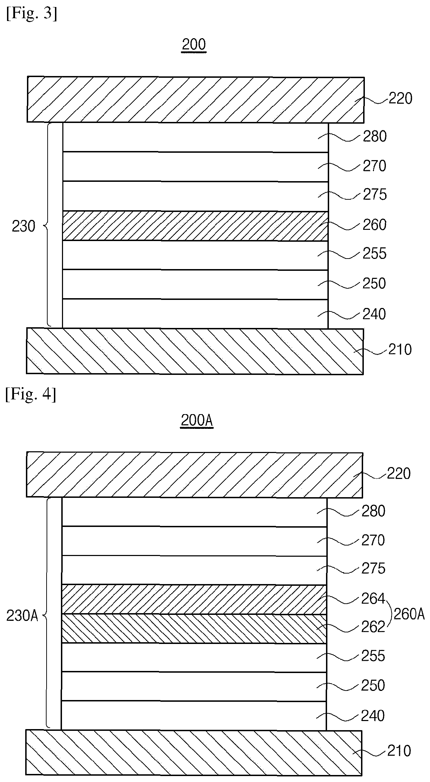

An emissive layer 230 is disposed on the first electrode 210. In one exemplary aspect, the organic emissive layer 230 0 may have a single-layered structure of an emitting material layer (EML). Alternatively, the organic emissive layer 230 may have a multiple-layered structure of a hole injection layer (HIL), a hole transport layer (HTL), an electron blocking layer (EBL), an EML, a hole blocking layer (HBL), an electron transport layer (ETL), an electron injection layer (EIL) and/or a charge generation layer (CGL) (see, FIGS. 3-4 and 6-9).

In addition, the organic emissive layer 230 may have a single emitting unit, or may have multiple emitting units to form a tandem structure. The organic emissive layer may includes an organic compound having a triazine moiety substituted with an aromatic group and/or a hetero aromatic group and a carbazole moiety fused with an aromatic ring and a hetero aromatic ring and linked to the triazine moiety via an aromatic and/or a hetero aromatic linker, and in which at least one protium of the triazine moiety and/or the aromatic/hetero aromatic linker moiety is substituted with deuterium. The structure and function of the organic emissive layer 230 of the OLED 200 will be explained in more detail.

The second electrode 220 is disposed over the substrate 102 above which the organic emissive layer 230 is disposed. The second electrode 220 may be disposed over a whole display area and may include a conductive material with a relatively low work function value compared to the first electrode 210. The second electrode 220 may be a cathode. For example, the second electrode 230 may include, but is not limited to, aluminum (Al), magnesium (Mg), calcium (Ca), silver (Ag), alloy thereof or combination thereof such as aluminum-magnesium alloy (Al-Mg).

In addition, an encapsulation film 180 may be disposed over the second electrode 230 in order to prevent outer moisture from penetrating into the OLED 200. The encapsulation film 180 may have, but is not limited to, a laminated structure of a first inorganic insulating film 182, an organic insulating film 184 and a second inorganic insulating film 186.

Moreover, a polarizer may be attached to the encapsulation film 180 in order to decrease external light reflection. For example, the polarizer may be a circular polarizer. In addition, a cover window may be attached to the encapsulation film 180 or the polarizer. In this case, the substrate 102 and the cover window may have a flexible property, thus the organic light emitting display device 100 may be a flexible display device.

Now, we will explain the OLED including the organic compound in which a portion of the nuclear atoms is substituted with deuterium is introduced to the organic emissive layer 230 so as to improve its luminous efficiency and luminous life time. FIG. 3 is a schematic cross-sectional view illustrating an OLED having single emitting unit in accordance with a first aspect of the present disclosure. As illustrated in FIG. 3, the OLED 200 includes first and second electrodes 210 and 220 facing each other; and the organic emissive layer 230 disposed between the first and second electrodes 210 and 220 and having single emitting unit. In one exemplary aspect, the organic emissive layer 230 comprises a HIL 240, a HTL 250, an EML 260, an ETL 270 and an EIL 280 each of which may be disposed sequentially between the first and second electrodes 210 and 220. Alternatively, the organic emissive layer 230 may further comprise a first exciton blocking layer, *i.e.* an EBL 255 disposed between the HTL 250 and the EML 260 and/or a second exciton blocking layer, *i.e.* a HBL 275 disposed between the EML 260 and the ETL 270.

The first electrode 210 may be an anode that provides a hole into the EML 260. The first electrode 210 may include, but is not limited to, a conductive material having a relatively high work function value, for example, a transparent conductive oxide (TCO). In an exemplary aspect, the first electrode 210 may include, but is not limited to, ITO, IZO, ITZO, SnO, ZnO, ICO, AZO, and the like.

The second electrode 220 may be a cathode that provides an electron into the EML 260. The second electrode 220 may include, but is not limited to, a conductive material having a relatively low work function values, i.e., a highly reflective material such as Al, Mg, Ca, Ag, alloy thereof, combination thereof, and the like. As an example, each of the first electrode 210 and the second electrode 230 may have a thickness, but is not limited to, between about 30 nm to about 300 nm.

The HIL 240 is disposed between the first electrode 210 and the HTL 250 and improves an interface property between the inorganic first electrode 210 and the organic HTL 250. In one exemplary aspect, the HIL 240 may include, but is not limited to, 4,4'4"-Tris(3-methylphenylamino)triphenylamine (MTDATA), 4,4',4"-Tris(N,N-diphenyl-amino)triphenylamine (NATA), 4,4',4"-Tris(N-(naphthalene-1-yl)-N-phenyl-amino)triphenylamine (1T-NATA), 4,4',4"-Tris(N-(naphthalene-2-yl)-N-phenyl-amino)triphenylamine (2T-NATA), Copper phthalocyanine (CuPc), Tris(4-carbazoyl-9-yl-phenyl)amine (TCTA), N,N'-Diphenyl-N,N'-bis(1-naphthyl)-1,1'-biphenyl-4,4"-diamine (NPB; NPD), 1,4,5,8,9,11-Hexaazatriphenylenehexacarbonitrile (Dipyrazino[2,3-f:2'3'-h]quinoxaline-2,3,6,7,10,11-hexacarbonitrile; HAT-CN), 1,3,5-tris[4-(diphenylamino)phenyl]benzene (TDAPB), poly(3,4-ethylenedioxythiphene)polystyrene sulfonate (PEDOT/PSS), N-(biphenyl-4-yl)-9,9-dimethyl-N-(4-(9-phenyl-9H-carbazol-3-yl)phenyl)-9H-fluoren-2-amine and/or N,N'-diphenyl-N,N'-di[4-(N,N-diphenyl-amino)phenyl]benzidine (NPNPB). The HIL 240 may be omitted in compliance with a structure of the OLED 200.

The HTL 250 is disposed adjacently to the EML 260 between the first electrode 210 and the EML 260. In one exemplary aspect, the HTL 250 may include, but is not limited to, N,N'-Diphenyl-N,N'-bis(3-methylphenyl)-1,1'-biphenyl-4,4'-diamine (TPD), NPB (NPD), 4,4'-bis(N-carbazolyl)-1,1'-biphenyl (CBP), Poly[N,N'-bis(4-butylphenyl)-N,N'-bis(phenyl)-benzidine] (Poly-TPD), Poly[(9,9-dioctylfluorenyl-2,7-diyl)-co-(4,4'-(N-(4-sec-butylphenyl)diphenylamine))] (TFB), Di-[4-(N,N-di-p-tolyl-amino)-phenyl]cyclohexane (TAPC), 3,5-Di(9H-carbazol-9-yl)-N,N-diphenylaniline; DCDPA), N-(biphenyl-4-yl)-9,9-dimethyl-N-(4-(9-phenyl-9H-carbazol-3-yl)phenyl)-9H-fluoren-2-amine, N-(biphenyl-4-yl)-N-(4-(9-phenyl-9H-carbazol-3-yl)phenyl)biphenyl-4-amine and/or N-([1,1'-Biphenyl]-4-yl)-9,9-dimethyl-N-(4-(9-phenl-9H-carbazol-3-yl)phenyl)-9H-fluore n-2-amine. As an example, each of the HIL 240 and the HTL 250 may have a thickness of, but is not limited to, between about 5 nm to about 200 nm, preferably about 5 nm to about 100 nm.

The EML 260 comprises a host and a dopant where the substantial light emission is occurred. In an exemplary aspect, the EML 260 includes a first host and a first dopant doped with the first host. As an example, the EML 260 may emit green (G) color. We will explain the kind of the host and dopant in the EML 260 in more detail.

The ETL 270 and the EIL 280 may be disposed sequentially between the EML 260 and the second electrode 220. The ETL 270 includes a material having high electron mobility so as to provide electrons stably with the EML 260 by fast electron transportation.

In one exemplary aspect, the ETL 270 may comprise, but is not limited to, oxadiazole-based compounds, triazole-based compounds, phenanthroline-based compounds, benzoxazole-based compounds, benzothiazole-based compounds, benzimidazole-based compounds, triazine-based compounds, and the like.

As an example, the ETL 270 may comprise, but is not limited to, tris-(8-hydroxyquinoline aluminum) (Alq ₃), 2-biphenyl-4-yl-5-(4-t-butylphenyl)-1,3,4-oxadiazole (PBD), spiro-PBD, lithium quinolate (Liq), 1,3,5-Tris(N-phenylbenzimidazol-2-yl)benzene (TPBi), Bis(2-methyl-8-quinolinolato-N1,O8)-(1,1'-biphenyl-4-olato)aluminum (BAlq), 4,7-diphenyl-1,10-phenanthroline (Bphen), 2,9-Bis(naphthalene-2-yl)4,7-diphenyl-1,10-phenanthroline (NBphen), 2,9-Dimethyl-4,7-diphenyl-1,10-phenaathroline (BCP), 3-(4-Biphenyl)-4-phenyl-5-tert-butylphenyl-1,2,4-triazole (TAZ), 4-(Naphthalen-1-yl)-3,5-diphenyl-4H-1,2,4-triazole (NTAZ), 1,3,5-Tri(p-pyrid-3-yl-phenyl)benzene (TpPyPB), 2,4,6-Tris(3'-(pyridin-3-yl)biphenyl-3-yl)1,3,5-triazine (TmPPPyTz), Poly[9,9-bis(3'-(N,N-dimethyl)-N-ethylammonium)-propyl)-2,7-fluorene]-alt-2,7-(9,9-dioctylfluorene)] (PFNBr), tris(phenylquinoxaline) (TPQ), Diphenyl-4-triphenylsilyl-phenylphosphine oxide (TSPO1), 2-[4-(9,10-Di-2-naphthalen2-yl-2-anthracen-2-yl)phenyl]-1-phenyl-1H-benzimidazole (ZADN) and combination thereof.

The EIL 280 is disposed between the second electrode 220 and the ETL 270, and can improve physical properties of the second electrode 220 and therefore, can enhance the lifetime of the OLED 200. In one exemplary aspect, the EIL 280 may comprise, but is not limited to, an alkali metal halide and/or alkaline earth metal halide such as LiF, CsF, NaF, BaF ₂ and the like, and/or an organic metal compound such as lithium quinolate, lithium benzoate, sodium stearate, and the like. As an example, each of the ETL 270 and the EIL 280 may have a thickness, but is not limited to, between about 10 to about 200 nm, preferably about 10 nm to about 100 nm.

When holes are transferred to the second electrode 220 via the EML 260 and/or electrons are transferred to the first electrode 210 via the EML 260, the OLED 200 may have short lifetime and reduced luminous efficiency. In order to prevent these phenomena, the OLED 200 in accordance with this embodiment of the present disclosure may have at least one exciton blocking layer adjacent to the EML 260.

For example, the OLED 200 includes the EBL 255 between the HTL 250 and the EML 260 so as to control and prevent electron transfers. In one exemplary aspect, the EBL 255 may comprise, but is not limited to, TCTA, Tris[4-(diethylamino)phenyl]amine, N-(biphenyl-4-yl)-9,9-dimethyl-N-(4-(9-phenyl-9H-carbazol-3-yl)phenyl)-9H-fluorene-2 -amine, TAPC, MTDATA, 1,3-Bis(carbazol-9-yl)benzene (mCP), 3,3'-bis(N-carbazolyl)-1,1'-biphenyl (mCBP), CuPc, N,N'-bis[4-(bis(3-methylphenyl)amino)phenyl]-N,N'-diphenyl-[1,1'-biphenyl]-4,4'-dia mine (DNTPD), TDAPB, DCDPA and/or 2,8-bis(9-phenyl-9H-carbazol-3-yl)dibenzo[b,d]thiophene.

In addition, the OLED 200 may further include the HBL 275 as a second exciton blocking layer between the EML 260 and the ETL 270 so that holes cannot be transferred from the EML 260 to the ETL 275. In one exemplary aspect, the HBL 275 may comprise, but is not limited to, oxadiazole-based compounds, triazole-based compounds, phenanthroline-based compounds, benzoxazole-based compounds, benzothiazole-based compounds, benzimidazole-based compounds, and triazine-based compounds each of which can be used in the ETL 270.

For example, the HBL 275 may comprise a compound having a relatively low HOMO energy level compared to the luminescent materials in EML 260. The HBL 275 may comprise, but is not limited to, BCP, BAlq, Alq ₃, PBD, spiro-PBD, Liq, Bis-4,5-(3,5-di-3-pyridylphenyl)-2-methylpyrimidine (B3PYMPM), DPEPO, 9-(6-(9H-carbazol-9-yl)pyridine-3-yl)-9H-3,9'-bicarbazole, TSPO1 and combination thereof.

As described above, the EML 360 may comprise the firs host and the first dopant. For example, the first host may have the following structure of Chemical Formula 1:

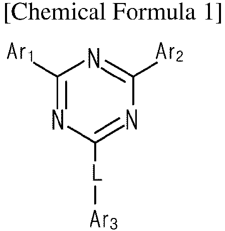

wherein each of Ar₁ and Ar₂ is independently an unsubstituted or substituted C ₆~C ₃₀ aromatic group or an unsubstituted or substituted C ₃~C₃₀ hetero aromatic group; L is an unsubstituted or substituted C ₆~C ₃₀ arylene group or an unsubstituted or substituted C₃~C₃₀ hetero arylene group; Ar₃ has the following structure of Chemical Formula 2, wherein at least one of nuclear atoms of an aromatic ring and a hetero aromatic ring constituting at least one of Ar ₁, Ar₂ and L is substituted with deuterium:

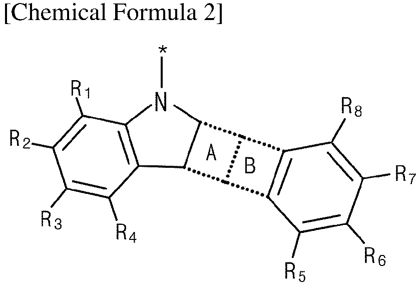

wherein each of R ₁ to R ₈ is independently selected from the group consisting of hydrogen, an halogen atom, a cyano group, a nitro group, an amino group, a C ₁~C₁₀ alkyl halide group, an unsubstituted or substituted linear or branched C ₁~C ₁₀ alkyl group, an unsubstituted or substituted C ₁~C ₁₀ alkoxy group, an unsubstituted or substituted C ₆~C ₃₀ aromatic group and an unsubstituted or substituted C ₃~C ₃₀ hetero aromatic group; A is a fused aromatic group having the following structure of Chemical Formula 3A; and B is fused hetero aromatic group having the following structure of Chemical Formula 3B:

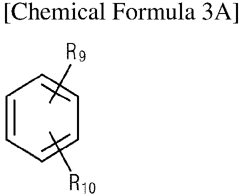

wherein each of R ₉ and R ₁₀ is independently selected from the group consisting of hydrogen, an halogen atom, a cyano group, a nitro group, an amino group, a C ₁~C ₁₀ alkyl halide group, an unsubstituted or substituted linear or branched C ₁~C ₁₀ alkyl group, an unsubstituted or substituted C ₁~C ₁₀ alkoxy group, an unsubstituted or substituted C ₆~C ₃₀ aromatic group and an unsubstituted or substituted C ₃~C ₃₀ hetero aromatic group:

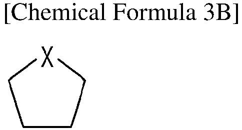

wherein X is oxygen (O), sulfur (S) or NR ₁₁, and wherein R ₁₁ is selected from the group consisting of protium, deuterium, an halogen atom, a cyano group, a nitro group, an amino group, a C ₁~C ₁₀ alkyl halide group, an unsubstituted or substituted linear or branched C ₁~C ₁₀ alkyl group, an unsubstituted or substituted C ₁~C ₁₀ alkoxy group, an unsubstituted or substituted C ₆~C ₃₀ aromatic group and an unsubstituted or substituted C ₃~C ₃₀ hetero aromatic group.

As used herein, the term "unsubstituted" means that a group has only hydrogen as a substituent, and in this case, hydrogen comprises protium.

As used the term "substituted" herein, the substitution group comprises, but is not limited to, unsubstituted or halogen-substituted C ₁-C ₂₀ alkyl, unsubstituted or halogen-substituted C ₁-C ₂₀ alkoxy, halogen, cyano, -CF ₃, a hydroxyl group, a carboxylic group, a carbonyl group, an amino group, a C ₁-C ₁₀ alkyl amino group, a C ₆-C ₃₀ aryl amino group, a C ₃-C ₃₀ hetero aryl amino group, a C ₆-C ₃₀ aryl group, a C ₃ - C₃₀ hetero aryl group, a nitro group, a hydrazyl group, a sulfonate group, a C ₁-C ₂₀ alkyl silyl group, a C ₆-C ₃₀ aryl silyl group and a C ₃-C ₃₀ hetero aryl silyl group.

As used herein, the term 'hetero" in such as "a hetero aromatic ring", "a hetero cycloalkyene group", "a hetero arylene group", "a hetero aryl alkylene group", " a hetero aryl oxylene group", "a hetero cycloalkyl group", "a hetero aryl group", "a hetero aryl alkyl group", "a hetero aryloxyl group", "a hetero aryl amino group" means that at least one carbon atom, for example 1-5 carbons atoms, constituting an aromatic ring or an alicyclic ring is substituted with at least one hetero atom which may be selected from the group consisting of N, O, S, P and combination thereof.

In one exemplary aspect, the C ₆~C ₃₀ aromatic group in each of Ar₁ and/or Ar₂ in Chemical Formula 1, R ₁ to R ₈ in Chemical Formula 2 and R₉ to R₁₁ in Chemical Formulae 3A and 3B is a group which has one or more C ₆-C ₃₀ aryl groups therein, and which may comprise a C ₆-C ₃₀ aryl group, an alkyl group substituted with one or more C ₆-C ₃₀ aryl groups, a C ₆-C ₃₀ aryloxyl group, an amino group substituted with one or more C ₆-C ₃₀ aryl groups, and combination thereof. The hetero aromatic group in each of Ar ₁ and/or Ar ₂ in Chemical Formula 1, R ₁ to R ₈ in Chemical Formula 2 and R₉ to R ₁₁ in Chemical Formulae 3A and 3B is a group which has one or more C ₃ - C₃₀ hetero aryl groups therein, and which may comprise a C ₃-C ₃₀ hetero aryl group, an alkyl group substituted with one or more C ₃-C ₃₀ hetero aryl groups, a C ₃-C ₃₀ hetero aryloxyl group, an amino group substituted with one or more C ₃-C ₃₀ hetero aryl groups, and combination thereof.

In one exemplary aspect, the C ₆-C ₃₀ aryl group in each of Ar₁ and/or Ar ₂ in Chemical Formula 1, R ₁ to R ₃ in Chemical Formula 2 and R₉ to R ₁₁ in Chemical Formulae 3A and 3B may comprise independently, but is not limited to, an unfused or fused aryl group such as phenyl, biphenyl, terphenyl, naphthyl, anthracenyl, pentalenyl, indenyl, indenoindenyl, heptalenyl, biphenylenyl, indacenyl, phenalenyl, phenanthrenyl, benzophenanthrenyl, dibenzophenanthrenyl, azulenyl, pyrenyl, fluoranthenyl, triphenylenyl, chrysenyl, tetraphenylenyl, tetracenyl, pleiadenyl, pycenyl, pentaphenylenyl, pentacenyl, fluorenyl, indenofluorenyl and spiro-fluorenyl.

In another exemplary aspect, the C ₃-C ₃₀ hetero aryl group in each of Ar₁ and/or Ar₂ in Chemical Formula 1, R ₁ to R ₈ in Chemical Formula 2 and R ₉ to R ₁₁ in Chemical Formulae 3A and 3B may comprise independently, but is not limited to, an unfused or fused hetero aryl group such as pyrrolyl, pyridinyl, pyrimidinyl, pyrazinyl, pyridazinyl, triazinyl, tetrazinyl, imidazolyl, pyrazolyl, indolyl, iso-indolyl, indazolyl, indolizinyl, pyrrolizinyl, carbazolyl, benzocarbazolyl, dibenzocarbazolyl, indolocarbazolyl, indenocarbazolyl, benzofurocarbazolyl, benzothienocarbazolyl, carbolinyl, quinolinyl, isoquinolinyl, phthlazinyl, quinoxalinyl, cinnolinyl, quinazolinyl, quinolizinyl, purinyl, benzoquinolinyl, benzoiso-quinolinyl, benzoquinazolinyl, benzoquinoxalinyl, acridinyl, phenazinyl, phenoxazinyl, phenothiazinyl, phenanthrolinyl, perimidinyl, phenanthridinyl, phtheridinyl, naphthyridinyl, furanyl, pyranyl, oxazinyl, oxazolyl, oxadiazolyl, triazolyl, dioxinyl, benzofuranyl, dibenzofuranyl, thiopyranyl, xanthenyl, chromenyl, iso-chromenyl, thioazinyl, thiophenyl, benzothiophenyl, dibenzothiophenyl, difuropyrazinyl, benzofurodibenzofuranyl, benzothienobenzothiophenyl, benzothienodibenzothiophenyl, benzothienobenzofuranyl, benzothienodibenzofuranyl, xanthene-linked spiro acridinyl, dihydroacridinyl substituted with at least one C ₁-C ₁₀ alkyl and N-substituted spiro fluorenyl.

As an example, each of the aromatic group and/or the hetero aromatic group in each of Ar ₁ and/or Ar ₂ in Chemical Formula 1, R ₁ to R ₈ in Chemical Formula 2 and R₉ to R ₁₁ in Chemical Formulae 3A and 3B may have independently one to three aromatic or hetero aromatic rings. For example, such an aromatic and/or a hetero aromatic group may comprise, but is not limited to, phenyl, biphenyl, naphthyl, anthracenyl, pyrrolyl, triazinyl, imidazolyl, pyrazolyl, pyridinyl, pyrazinyl, pyrimidinyl, pyridazinyl, furanyl, benzofuranyl, dibenzofuranyl, thiophenyl, benzothiophenyl, dibenzothiophenyl, carbazolyl, acridinyl, carbolinyl, phenazinyl, phenoxazinyl and/or phenothiazinyl.

As defined in Chemical Formulae 1 to 3B, the organic compound of the present disclosure includes a triazine moiety having a strong electron acceptor property, a carbazole moiety having the structure of Chemical Formula 2 and fused with an aromatic ring and a hetero aromatic ring having strong electron donor property, and an aromatic or a hetero aromatic linker L between the triazine moiety and the carbazole moiety. The carbazole moiety fused with an aromatic ring and a hetero aromatic ring may form, but is not limited to, an indolocarbazole moiety, a benzofurocarbazole moiety or a benzothienocarbazole moiety.

For example, when L is a C ₆-C ₃₀ arylene group, the L may comprise, but is not limited to, phenylene, biphenylene, terphenylene, tetraphenylene, indenylene, naphthylene, zulenylene, indacenylene, acenaphthylene, fluorenylene, spirofluorenylene, phenalenylene, phenanthrenylene, anthracenylene, fluoranthrenylene, triphenylenylene, pyrenylene, chrysenylene, naphthacenylene, pycenylene, perylenylene, pentaphenylen and hexacenylene.

Alternatively, when L is a C ₃-C ₃₀ hetero arylene group, the L may comprise, but is not limited to, pyrrolylene, imidazolylene, pyrazolylene, pyridinylene, pyrazinylene, pyrimidinylene, pyridazinylene, isoindolylene, indolylene, indazolylene, purinylene, quinolinylene, isoquinolinylene, benzoquinolinylene, phthalazinylene, naphthyridinylene, quinoxalinylene, quinazolinylene, benzoisoquinolinylene, benzoquinazolinylene, benzoquinoxalinylene, cinnolinylene, phenanthridinylene, acridinylene, phenanthrolinylene, phenazinylene, benzoxazolylene, benzimidazolylene, furanylene, benzofuranylene, thiophenylene, benzothiophenylene, thiazolylene, isothiazolylene, benzothiazolylene, isoxazolylene, oxazolylene, triazolyene, tetrazolylene, oxadiazolylene, triazinylene, benzofuranylene, dibenzofuranylene, benzofurodibenzofuranylene, benzothienofuranylene, benzothienobenzofuranylene, benzothienodibenzofuranylene, benzothienobenzothiophenylene, benzothienodibenzothiophenylene, carbazolyene, benzocarbazolylene, dibenzocarbazolylene, indolocarbazolylne, indenocarbazolylene, beznofurocarbazolylene, benzothienocarbazolylene, imidazopyrimidinylene and imidazopyridinylene.

In one exemplary aspect, when the number of the aromatic and/or the hetero aromatic ring constituting L becomes large, the conjugation structure within the whole organic molecule is too long, and therefore, the organic compound may have too narrow energy bandgap. Accordingly, L may have one or two, preferably one aromatic and/or hetero aromatic ring. With regard charge injection and transfer property, L may be a 5-membererd ring, a 6-memered ring or 7-membererd ring, and particularly a 6-membered ring. For example, L may comprise, but is not limited to, phenylene, biphenylene, pyrrolylene, imdiazolylene, pyrazolylene, pyridylene, pyrazinlylene, pyrimidylene, pyrazinylene, pyridazinlylene, furanylene and thiophenylene.

As defined in Chemical Formulae 1 to 3B, at least one of the nuclear atoms among Ar ₁ and Ar ₂ each of which is substituted to the triazine moiety with strong electron acceptor property and the aromatic/hetero aromatic ring constituting L is substituted with deuterium.

Compared to hydrogen atoms in the carbazole moiety fused with an aromatic ring and a hetero aromatic ring each of which has strong electron donor property, the hydrogen atoms in the group substituted to the triazine moiety having strong electron acceptor moiety, the triazine moiety and/or the aromatic/hetero aromatic linker L between the triazine moiety and the carbazole moiety are adjacent to plural nitrogen atoms having an electron affinity stronger than that of the hydrogen atom. Accordingly, compared to the acidity of the hydrogen atoms linked to the nuclear atoms constituting the carbazole moiety or the nuclear atoms in the aromatic or hetero aromatic ring fused with the carbazole moiety, the acidity of the hydrogen atoms linked to the nuclear atoms in Ar ₁ and Ar ₂ substituted to the triazine moiety and/or the nuclear atoms in the aromatic/hetero aromatic ring constituting L is high ( *i.e.,* lower pKa).

When organic compounds are substituted with deuterium, deuterium raw material such as d6-benzene or D ₂O is reacted with a protium-substituted compound having an entire carbon backbone molecule under acid or base catalysis condition. In this case, large amount of expensive deuterium raw materials must be used, which results in environmental pollution in synthetic process.

However, even in case of using the organic compound which substitutes only the protium, which has relatively high acidity (relatively low pKa), linked to the nuclear atom forming the aromatic and/or hetero aromatic group substituted to the triazine moiety and/or the aromatic/hetero aromatic ring constituting the linker with deuterium in accordance with the present disclosure, the organic compound can realize excellent luminous efficiency and luminous life time as organic compound in which the nuclear atoms in all aromatic/hetero aromatic rings constituting the entire molecule. In other words, substituting only the protium atoms linked to the nuclear atoms in the aromatic or hetero aromatic rings constituting the specific moiety with relatively high acidity (relatively low pKa) with deuterium atoms can prevent the protium atoms from being dissociated from the molecule, and therefore it is possible to improve an electrochemical stability of the molecule and to enhance the luminous efficiency and luminous life time of the molecule. Since it is not necessary to substitute the nuclear atoms in the whole aromatic or hetero aromatic rings constituting the backbone of the entire molecule with deuterium, it is possible to reduce the manufacturing cost by reducing the used amount of the expensive deuterium raw material, and to avoid environmental pollution problems caused by using a large amount deuterium. In other words, the OLED 200 including the organic compound in which contains selectively substituted deuterium linked to specific moieties or positions having substantially high acidity (low pKa) can significantly improve its luminous efficiency and luminous life time.

In one exemplary aspect, at least one of the nuclear atoms in the Ar ₁ moiety and Ar ₂ moiety each of which is substituted to the triazine moiety having strong electron acceptor property may be substituted with deuterium. In another exemplary aspect, at least one of the nuclear atoms in the aromatic or hetero aromatic linker L may be substituted with deuterium. On the contrary, the nuclear atoms in the Ar₃ moiety of the fused carbazole moiety having strong electron donor property.

In one exemplary aspect, the Ar₃ moiety of the fused carbazole moiety may have any of the following structure of Chemical Formulae 4A to 4F. As an example, Ar ₃ may have, but is not limited to, the following structure of Chemical Formula 4C or 4D:

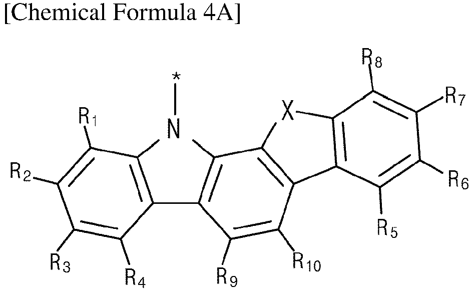

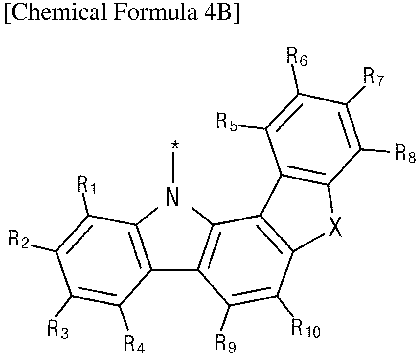

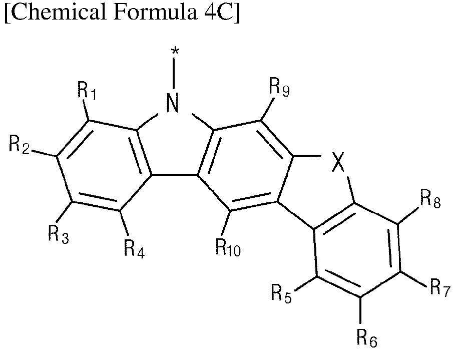

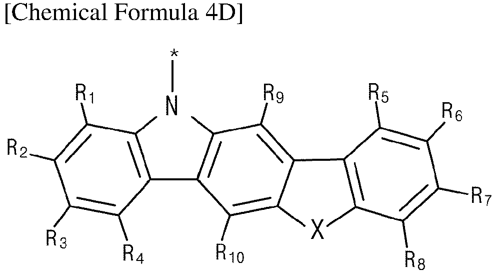

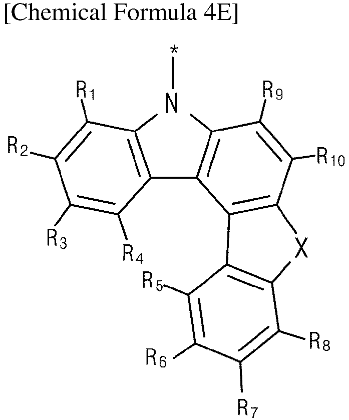

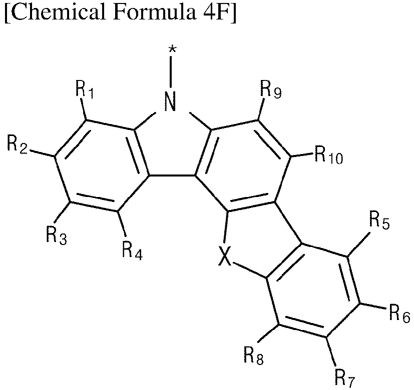

wherein each of R ₁ to R ₁₀ and X is identical to as defined in Chemical Formulae 2, 3A and 3B.

More particularly, the organic compound having the structure of Chemical Formula 1 may comprise any one having the following structure of Chemical Formula 5:

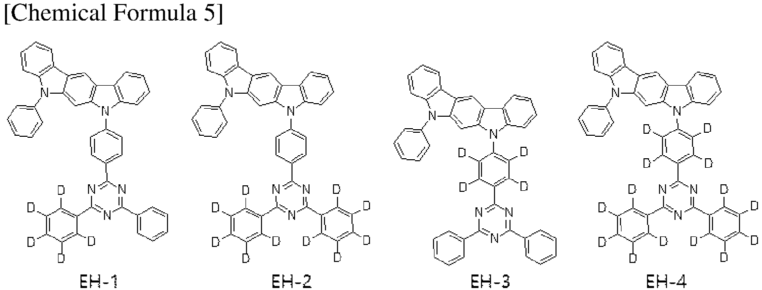

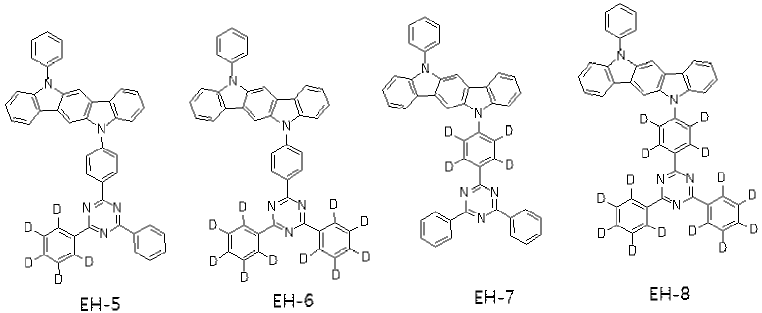

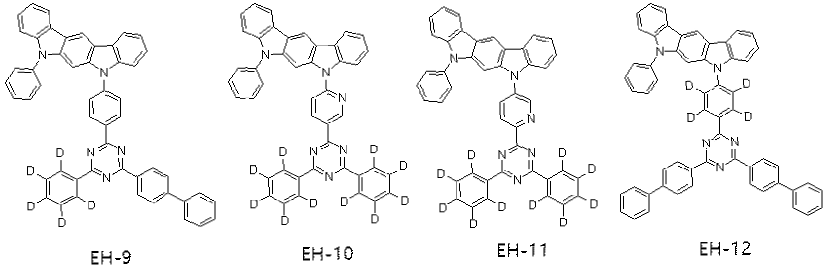

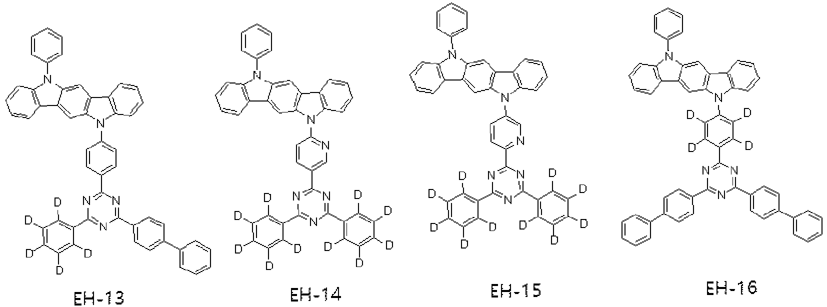

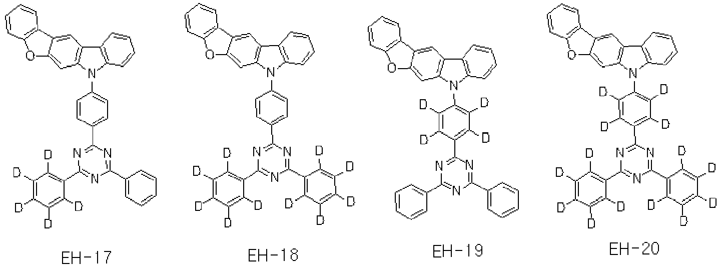

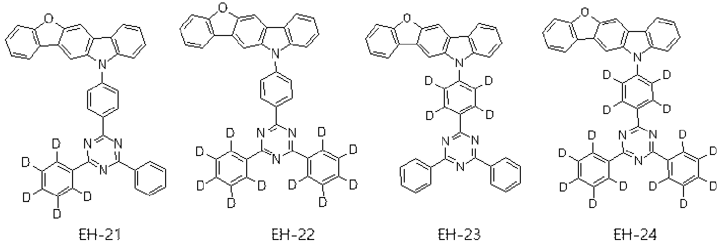

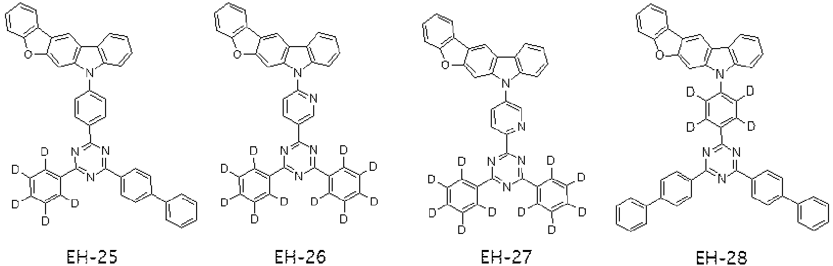

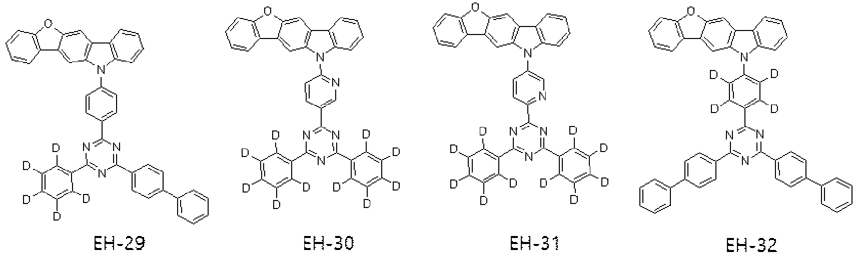

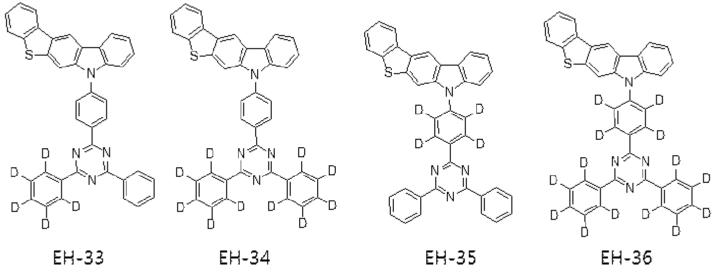

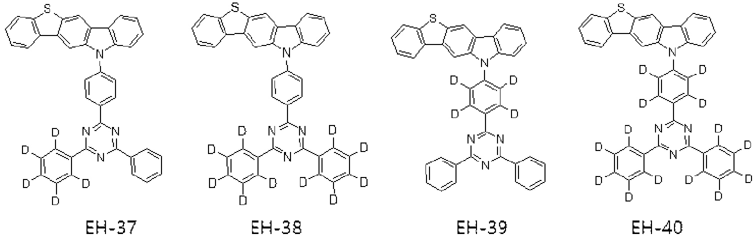

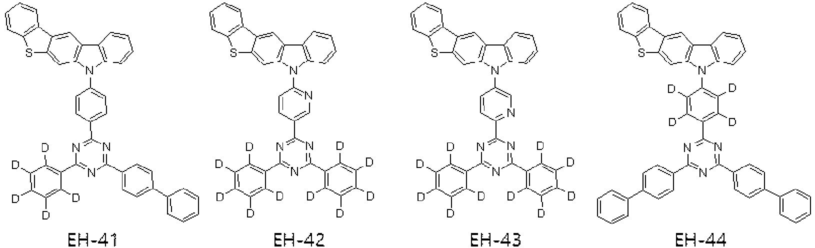

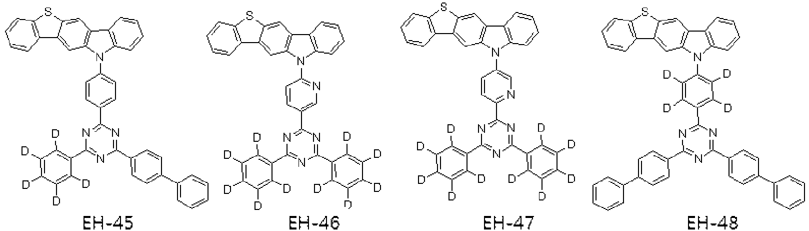

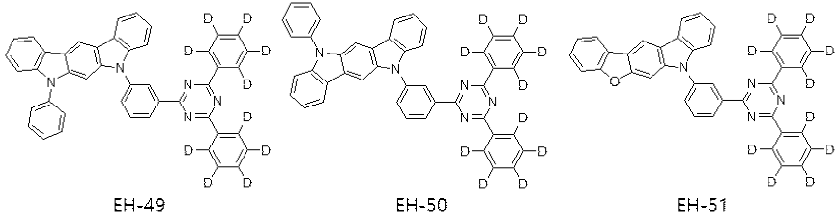

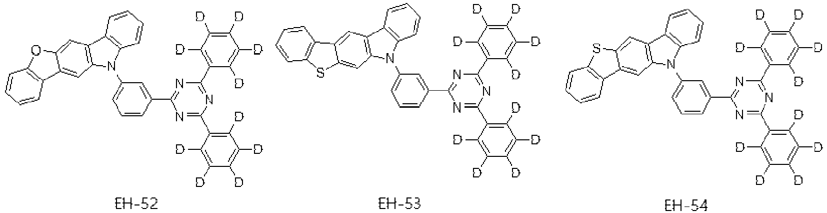

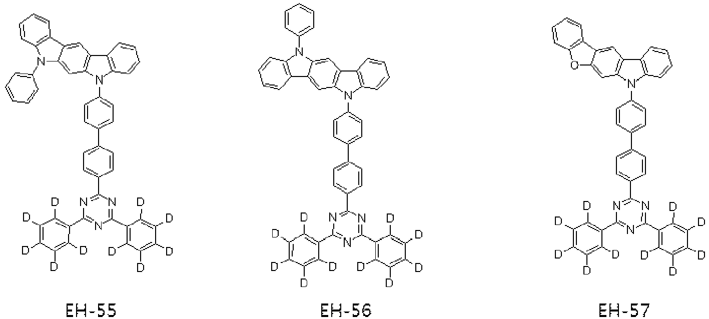

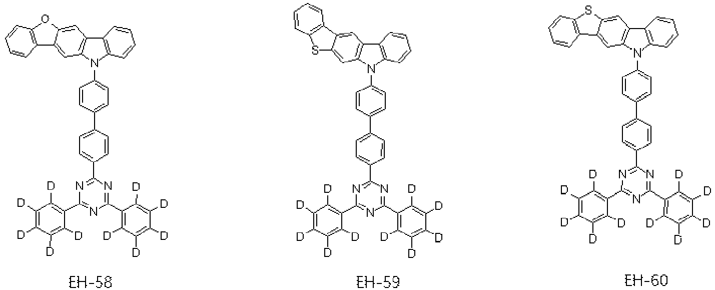

As an example, any one having the structure of Chemical Formula 1 to 5 may be used as a first host in the EML 260. For example, the organic compound having the structure of Chemical Formula 1 to 5 may be green host in the EML 260. Alternatively, the EML 260 may further comprise other green host as a second host. The second host that can be used with the organic compound having the structure of Chemical Formulae 1 to 5 may comprise a p-type host with high affinity property to holes.

In one exemplary aspect, the host (green host) which can be used with the organic compound having the structure of Chemical Formulae 1 to 5 may comprise, but is not limited to, 9,9'-Diphenyl-9H,9'H-3,3'-bicarbazole (BCzPh), CBP, 1,3,5-Tris(carbazole-9-yl)benzene (TCP), TCTA, 4,4'-Bis(carbazole-9-yl)-2,2'-dimethylbipheyl (CDBP), 2,7-Bis(carbazole-9-yl)-9,9-dimethylfluorene (DMFL-CBP), 2,2',7,7'-Tetrakis(carbazole-9-yl)-9,9-spiorofluorene (Spiro-CBP), DPEPO, 4'-(9H-carbazol-9-yl)biphenyl-3,5-dicarbonitrile (PCzB-2CN), 3'-(9H-carbazol-9-yl)biphenyl-3,5-dicarbonitrile (mCzB-2CN), 3,6-Bis(carbazole-9-yl)-9-(2-ethyl-hexyl)-9H-carbazole (TCz1), and the like.

The first dopant in the EML 260 may be green dopant. For example, the first dopant which can be emit green color may comprise phorphorescent green dopant of a metal complex having the follwoing structure of Chemical Formula 6A or Chemical Formula 6B:

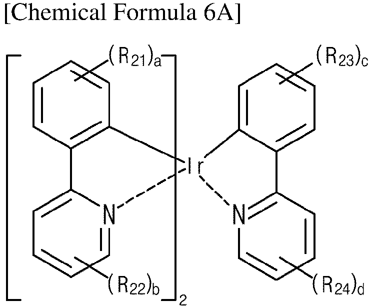

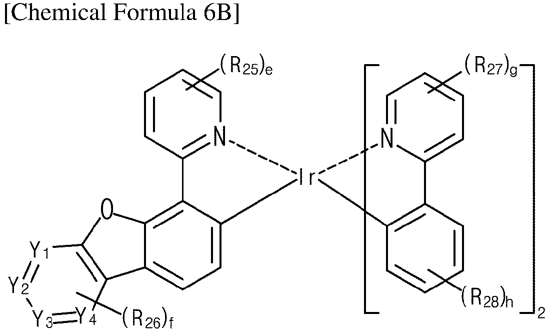

wherein each of R ₂₁ to R ₂₈ is independently protium, deuterium, a halogen atom, a C ₁~C ₆ alkyl group, a C ₃~C ₆ cyclo alkyl group, a C ₆~C ₁₀ aryl group or a C ₃~C ₁₀ hetero aryl group; each of a, c, d, e, g and h is indepenelty an integer of 0 to 4, and each of b and f is independently an integer of 0 to 3; and each of Y₁ to Y₄ is independently nitronge (N) or CR ₂₉, wherein R ₂₉ protium, deuterium, a halogen atom, a C ₁~C ₆ alkyl group, a C ₃~C ₆ cyclo alkyl group, a C ₆~C ₁₀ aryl group or a C ₃~C ₁₀ hetero aryl group.

As an example, the first dopant as the green dopant including the metal complex having the structure of Chemical Formula 6A or Chemical Formula 6B may comprise, but is not limited to, [Bis(2-phenylpyridine)](pyridyl-2-benzofuro[2,3-b]pyridine)iridium, fac-Tris(2-phenylpyridine)iridium(III) (fac-Ir(ppy) ₃), Bis(2-phenylpyridine)(acetylacetonate)iridium(III) (Ir(ppy) ₂(acac)), Tris[2-(p-tolyl)pyridine]iridium(III) (Ir(mppy) ₃), Bis(2-(naphthalene-2-yl)pyridine)(acetylacetonate)iridium(III) (Ir(npy) ₂acac), Tris(2-phenyl-3-methyl-pyridine)iidium (Ir(mppy) ₃), fac-Tris(2-(3-p-xylyl)phenyl)pyridine iridium(III) (TEG), and the like.

For example, the contents of the first dopant in the EML 260 may be between about 1 wt% to about 50 wt%, preferably about 1 wt% to about 30 wt%. The EML 260 may have a thinkness, but is not limited to, between about 10 nm to about 200 nm, preferably about 20 nm to about 100 nm, and more preferably about 20 nm to about 50 nm.

In the above aspet, the EML 260 emit green color. Unlike that aspect, the EML 260 may emit red-green (RD) or yellow-green (YG) color. FIG. 4 is a schematic cross-sectional view illustrating an OLED having single emitting unit in accordance with a second aspect of the present disclosure. Similar to the OLED 200 illustrated in FIG. 3, the OLED 200A comprise the first and second electrodes 210 and 220 facing each other and an organic emissive layer 230A having single emitting unit between the first and second electrode 210 and 220. The organic emissive layer 230A may comprise the HIL 240, the HTL 250, an EML 260A, the ETL 270 and the EIL 280. Alternatively, the organic emissive layer 230A may further comprise the EBL 255 between the HTL 250 and the EML 260A and/or the HBL 275 between the EML 260A and the ETL 270. The configuration of the organic emissive layer 230A is substantially the same of the configuration of the organic emissive layer 230 except the EML 260A.

In the second aspect of the present disclosure, the EML 260A comprises a lower EML 262 disposed between the EBL 255 and the HBL 275 and an upper EML 264 disposed between the upper EML 262 and the HBL 275. One of the lower EML 262 and the upper EML 264 may be green EML including the organic compound having the structure of Chemical Formulae 1 to 5. The other of the lower EML 262 and the upper EML 264 may be red EML and/or yellow EML. Hereinafter, the EML 260 where the lower EML 262 is green EML and the upper EML 264 is red and/or yellow EML will be explained in detail.

The lower EML 262 includes the organic compound having the strucutre of Chemical Formulae 1 to 5. As an example, the lower EML 262 may comprise the first host and the first dopant, and the first host comprises the organic compound having the structure of Chemical Formulae 1 to 5. The frist doapnt may be green dopant. The first dopant may comprise the metal complex having the strucutre of Chemical Formula 6A or Chemical Formula 6B. As an example, the first dopant in the lower EML 262 may comprise, but is not limited to, [Bis(2-phenylpyridine)](pyridyl-2-benzofuro[2,3-b]pyridine)iridium, fac-Ir(ppy) ₃, Ir(ppy) ₂(acac), Ir(mppy) ₃, Ir(npy) ₂acac, Ir(mppy) ₃, TEG, and the like.

If necessary, the lower EML 262 may further comprise the seocnd host which can be used with the first host. The seocnd host may comprise, but is not limited to, BCzPh, CBP, TCP, TCTA, CDBP, DMFL-CBP, Spiro-CBP, DPEPO, PCzB-2C), mCzB-2CN, TCz1, and the like.

The upper EML 264 may comprise a third host and a seocnd dopant. In one exemplary aspect, the third host may be red host and the second dopant may be red dopant.

The third host which can be used as the red host may comprise the seocnd host as desdribed above. Alternatively, the third host which can be used as the red host may comprise, but is not limited to, Bis(2-hydroxylphenyl)-pyridine)beryllium (Bepp ₂), Bis(10-hydroxylbenzo[h] quinolinato)beryllium (Bebq ₂), 1,3,5-Tris(1-pyrenyl)benzene (TPB3), and the like.

The second dopant which can be used as the red dopant may comprise, but is not limited to, a metal complex having the structure of Chemcial Formula 7A or Chemical Formula 7B:

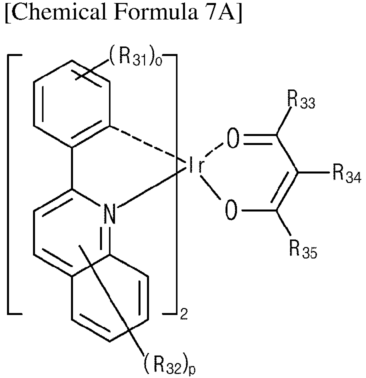

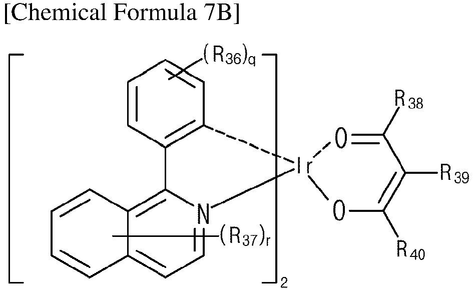

wherein each of R₃₁, R₃₂, R₃₆ and R₃₇ is independently protium, deuterium, a halogen atom, a C ₁~C ₆ alkyl group, a C ₃~C ₆ cyclo alkyl group, a C ₆~C ₁₀ aryl group or a C ₃~C ₁₀ hetero aryl group; each of o and q is independently an integer of 0 to 4, and each of p and r is independently an integer of 0 to 6; and each of R₃₃ to R₃₅ and R ₃₈ to R ₄₀ is independently protium, deuterium or a C ₁~C ₆ alkyl group.

As an example, the second dopant as the red dopant including the metal complex having the strucutre of Chemical Formula 7A or Chemical Formula 7B may comprise, but is not limited to, [Bis(2-(4,6-dimethyl)phenylquinoline)](2,2,6,6-tetramethylheptane-3,5-dionate)iridiu m(III), Bis[2-(4-n-hexylphenyl)quinoline](acetylacetonate)iridium(III) (Hex-Ir(phq) ₂ (acac)), Tris[2-(4-n-hexylphenyl)quinoline]iridium(III) (Hex-Ir(phq) ₃), Tris[2-phenyl-4-methylquinoline]iridium(III) (Ir(Mphq) ₃), Bis(2-phenylquinoline)(2,2,6,6-tetramethylheptene-3,5-dionate)iridium(III) (Ir(dpm)PQ ₂), (Bis(phenylisoquinoline)(2,2,6,6-tetramethylheptene-3,5-dionate)iridium(III) (Ir(dpm)(piq) ₂), Bis[(4-n-hexylphenyl)isoquinoline](acetylacetonate)iridium(III) (Hex-Ir(piq) ₂(acac)), Tris[2-(4-n-hexylphenyl)quinoline]iridium(III) (Hex-Ir(piq) ₃), Tris(2-(3-methylphenyl)-7-methyl-quinolato)iridium (Ir(dmpq) ₃), Bis[2-(2-methylphenyl)-7-methyl-quinoline](acetylacetonate)iridium(III) (Ir(dmpq) ₂ (acac)), Bis[2-(3,5-dimethylphenyl)-4-methyl-quinoline](acetylacetonate)iridium(III) (Ir(mphmq) ₂(acac)), and the like.

Alternatively, the third host in the upper EML 264 may be yellow host and the second dopant may be yellow dopant. As an example, the third host which can be yellow host may be identical to the second host of the green host as described above or to the third host of the red host as described above.

The second dopant which can be used as the yellow dopant may comprise, but is not limited to, 5,6,11,12-Tetraphenylnaphthalene (Rubrene), 2,8-Di-tert-butyl-5,11-bis(4-tert-butylphenyl)-6,12-diphenyltetracene (TBRb), Bis(2-phenylbenzothiazolato)(acetylacetonate)irdium(III) (Ir(BT) ₂(acac)), Bis(2-(9,9-diethytl-fluoren-2-yl)-1-phenyl-1H-benzo[d]imdiazolato)(acetylacetonate)ir idium(III) (Ir(fbi) ₂(acac)), Bis(2-phenylpyridine)(3-(pyridine-2-yl)-2H-chromen-2-onate)iridium(III) (fac-Ir(ppy) ₂Pc), Bis(2-(2,4-difluorophenyl)quinoline)(picolinate)iridium(III) (FPQIrpic), and the like.

For example, when the upper EML 264 includes the third host and the second dopant, the conetns of the second dopant in the upper EML 264 may be between about 1 wt% to about 50 wt%, preferably about 1 wt% to about 30 wt%. Each of the lower EML 262 and the upper EML 264 may have a thickness, but is not limited to, beteen about 10 nm to about 100 nm, preferably about 10 nm to about 50 nm.

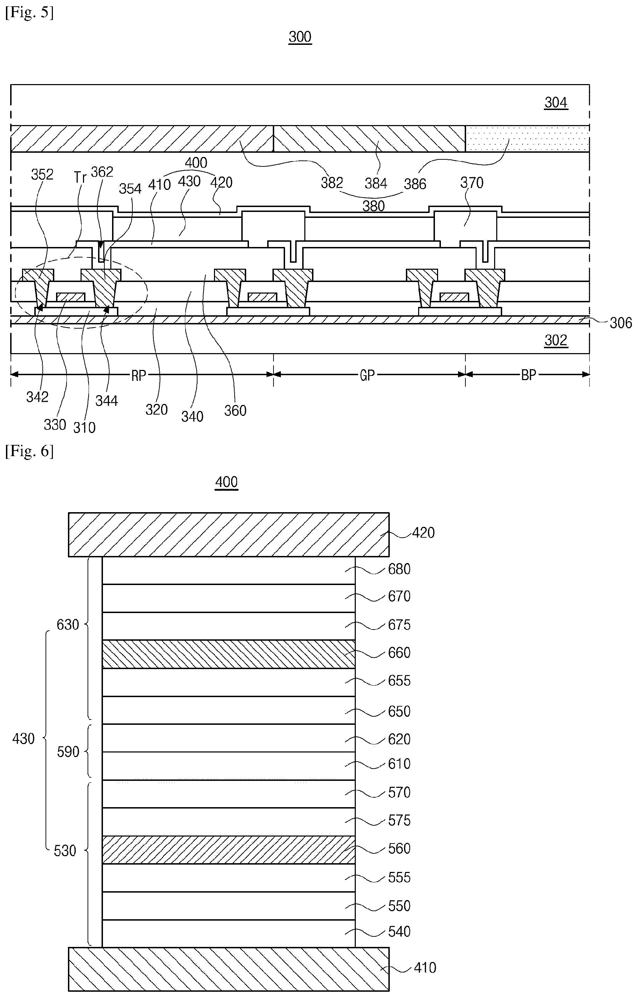

In FIGS. 2 to 4, the organic light emitting diode and the light emitting dispaly device that includes single emitting unit emitting green or yellow green. Unlikely, an organic light emititng diode can implement a full-color display incluidhng white color. FIG. 5 is a schematic cross-sectional view illustrating an organic light emitting display device in accordance with a second aspect of the present disclosure.

As illustrated in FIG. 5, the organic light emitting display device 300 comprises a first substrate 302 that defines each of a red pixel RP, a green pixel GP and a blue pixel BP, a second substrate 304 facing the first substrate 302, a thin film transistor Tr over the first substrate 302, an OLED 400 disposed between the first and second substrates 302 and 304 and emitting white (W) light and a color filter layer 380 disposed between the OLED 400 and the second substrate 304.

Each of the first and second substrates 302 and 304 may include, but is not limited to, glass, flexible material and/or polymer plastics. For example, each of the first and second substrates 302 and 304 may be made of PI, PES, PEN, PET, PC and combination thereof. The first substrate 302, over which a thin film transistor Tr and the OLED 400 are arranged, forms an array substrate.

A buffer layer 306 may be disposed over the first substrate 302, and the thin film transistor Tr is disposed over the buffer layer 306 correspondingly to each of the red pixel RP, the green pixel GP and the blue pixel BP. The buffer layer 306 may be omitted.

A semiconductor layer 310 is disposed over the buffer layer 306. The semiconductor layer 310 may be made of oxide semiconductor material or polycrystalline silicon.

A gate insulating layer 320 including an insulating material, for example, inorganic insulating material such as silicon oxide (SiO ₓ) or silicon nitride (SiN ₓ) is disposed on the semiconductor layer 310.

A gate electrode 330 made of a conductive material such as a metal is disposed over the gate insulating layer 320 so as to correspond to a center of the semiconductor layer 310. An interlayer insulting layer 340 including an insulating material, for example, inorganic insulating material such as silicon oxide (SiO ₓ) or silicon nitride (SiN ₓ), or an organic insulating material such as benzocyclobutene resin or photo-acryl, is disposed on the gate electrode 330.

The interlayer insulating layer 340 has first and second semiconductor layer contact holes 342 and 344 that expose both sides of the semiconductor layer 310. The first and second semiconductor layer contact holes 342 and 344 are disposed over opposite sides of the gate electrode 330 with spacing apart from the gate electrode 330.

A source electrode 352 and a drain electrode 354, which are made of a conductive material such as a metal, are disposed on the interlayer insulating layer 340. The source electrode 352 and the drain electrode 354 are spaced apart from each other with respect to the gate electrode 330, and contact both sides of the semiconductor layer 310 through the first and second semiconductor layer contact holes 342 and 344, respectively.

The semiconductor layer 310, the gate electrode 330, the source electrode 352 and the drain electrode 354 constitute the thin film transistor Tr, which acts as a driving element.

Although not shown in FIG. 5, a gate line GL and a data line DL, which cross each other to define a pixel region P, and a switching element Ts, which is connected to the gate line GL and the data line DL, is may be further formed in the pixel region P. The switching element Tr is connected to the thin film transistor Tr, which is a driving element. In addition, a power line PL is spaced apart in parallel from the gate line GL or the data line DL, and the thin film transistor Tr may further include a storage capacitor Cst configured to constantly keep a voltage of the gate electrode 330 for one frame (see, FIG. 1).

A passivation layer 360 is disposed on the source and drain electrodes 352 and 354 with covering the thin film transistor Tr over the whole first substrate 302. The passivation layer 360 has a drain contact hole 362 that exposes the drain electrode 354 of the thin film transistor Tr.

The OLED 400 is located over the passivation layer 360. The OLED 400 includes a first electrode 410 that is connected to the drain electrode 354 of the thin film transistor Tr, a second electrode 420 facing from the first electrode 410 and an organic emissive layer 430 disposed between the first and second electrodes 410 and 420.

The first electrode 410 formed for each pixel region may be an anode and may include a conductive material having relatively high work function value. For example, the first electrode 410 may include, ITO, IZO, ITZO, SnO, ZnO, ICO, AZO, and the like. Alternatively, a reflective electrode or a reflective layer (not shown) may be disposed under the first electrode 410. For example, the reflective electrode or the reflective layer (not shown) may include, but is not limited to, APC alloy.

A bank layer 370 is disposed on the passivation layer 360 in order to cover edges of the first electrode 410. The bank layer 370 exposes a center of the first electrode 410 corresponding to each of the red pixel RP, the green pixel GP and the blue pixel BP. The bank layer 370 may be omitted.

An organic emissive layer 430 including emitting units are disposed on the first electrode 410. As illustrated in FIGS. 6 to 9, the organic emissive layer 430 may include multiple emitting units 530, 530A, 630, 630A, 730, 730A, 830, 830A, 930, 930A and at least one charge generation layer 590, 790 and 890. Each of the emitting units 530, 530A, 630, 630A, 730, 730A, 830, 830A, 930, 930A includes an emitting material layer and may further include a HIL, a HTL, an EBL, a HBL, an ETL and/or an EIL.

The second electrode 420 is disposed over the first substrate 302 above which the organic emissive layer 430 is disposed. The second electrode 420 may be disposed over a whole display area, and may include a conductive material with a relatively low work function value compared to the first electrode 410, and may be a cathode. For example, the second electrode 420 may include, but is not limited to, aluminum (Al), magnesium (Mg), calcium (Ca), silver (Ag), alloy thereof or combination thereof such as aluminum-magnesium alloy (Al-Mg).

Since the light emitted from the organic emissive layer 430 is incident to the color filter layer 380 through the second electrode 420 in accordance with this exemplary aspect, the second electrode 420 has a thin thickness so that the light can be transmitted.

The color filter layer 380 is disposed over the OLED 400 and includes a red color filter 382, a green color filter 384 and a blue color filter 386 each of which is disposed correspondingly to the red pixel RP, the green pixel GP and the blue pixel BP, respectively. Although not shown in FIG. 5, the color filter layer 380 may be attached to the OLED 400 via an adhesive layer. Alternatively, the color filter layer 380 may be disposed directly on the OLED 400.

In addition, an encapsulation film (not shown) may be disposed over the second electrode 420 in order to prevent outer moisture from penetrating into the OLED 400. The encapsulation film (not shown) may have, but is not limited to, a laminated structure of a first inorganic insulating film (not shown), an organic insulating film (not shown) and a second inorganic insulating film (not shown) (See, 170 in FIG. 2). In addition, a polarizing plate (not shown) may be attached onto the second substrate 304 to reduce reflection of external light. For example, the polarizing plate (not shown) may be a circular polarizing plate.

In FIG. 5, the light emitted from the OLED 400 is transmitted through the second electrode 420 and the color filter layer 380 is disposed over the OLED 400. Alternatively, the light emitted from the OLED 400 is transmitted through the first electrode 410 and the color filter layer 380 may be disposed between the OLED 400 and the first substrate 302. In addition, a color conversion layer (not shown) may be formed between the OLED 400 and the color filter layer 380. The color conversion layer (not shown) may include a red color conversion layer (not shown), a green color conversion layer (not shown) and a blue color conversion layer (not shown) each of which is disposed correspondingly to each pixel (RP, GP and BP), respectively, so as to covert the white (W) color light to each of a red, green and blue color lights, respectively.

As described above, the white (W) color light emitted from the OLED 400 is transmitted through the red color filter 382, the green color filter 384 and the blue color filter 386 each of which is disposed correspondingly to the red pixel RP, the green pixel GP and the blue pixel BP, respectively, so that red, green and blue color lights are displayed in the red pixel RP, the green pixel GP and the blue pixel BP.

Now, we will describe an OLED that can be applied into the organic light emitting display device in accordance with this aspect. FIG. 6 is a schematic cross-sectional view illustrating an OLED having two emitting units in accordance with a third aspect of the present disclosure.

As illustrated in FIG. 6, the OLED 400 comprise first and second electrodes 410 and 420 facing each other and an organic emissive layer 430 disposed between the first and second electrodes 410 and 420. The organic emissive layer 430 comprises a first emitting unit 530 disposed between the first electrode 410 and 420, a second emitting unit 630 disposed between the first emitting unit 530 and the second electrode 420, and a charge generation layer (CGL) 590 disposed between the first and second emitting units 530 and 630.

The first electrode 410 may be an anode and include a conductive material having a relatively large work function values, for example, transparent conductive oxide (TCO) such as ITO, IZO, SnO, ZnO, ICO, AZO, and the like. The second electrode 420 may be a cathode and include a conductive material having a relatively small work function values such as Al, Mg, Ca, Ag, alloy thereof or combination thereof. As an example, each of the first and second electrodes 410 and 420 may be laminated with a thickness, but is not limited to, between about 30 nm to about 300 nm.

The first emitting unit 530 comprises a HIL 540, a first HTL (HTL1) 550, a first EML (EML1) 560 and a first ETL (ETL1) 570 each of which is disposed sequentially on the first electrode 410. Alternatively, the first emitting unit 430 may further comprise a first EBL (EBL1) 555 disposed between the HTL1 550 and the EML1 560 and/or a first HBL (HBL1) 575 disposed between the EML1 560 and the ETL1 570.

The second emitting unit 630 comprises a second HTL (HTL2) 650, a second EML (EML2) 660, a second ETL (ETL2) 670 and an EIL 670 each of which is disposed sequentially on the CGL 590. Alternatively, the second emitting unit 630 may further comprise a second EBL (EBL2) 655 disposed between the HTL2 650 and the EML2 660 and/or a second HBL (HBL2) 675 disposed between the EML2 660 and the ETL2 670.

One of the EML1 560 and the EML2 660 may comprise the organic compound having the structure of Chemical Formulae 1 to 5 to emit green color. The other of the EML1 560 and the EML2 660 may emit red (R) and/or blue (B) colors. Hereinafter, the OLED 400 where the EML2 660 comprises the organic compound having the structure of Chemical Formulae 1 to 5 will be explained.

The HIL 540 is disposed between the first electrode 410 and the HTL1 550 and improves an interface property between the inorganic first electrode 410 and the organic HTL1 550. In one exemplary embodiment, the HIL 540 include, but is not limited to, MTDATA, NATA, 1T-NATA, 2T-NATA, CuPc, TCTA, NPB(NPD), HAT-CN, TDAPB, PEDOT/PSS, N-(biphenyl-4-yl)-9,9-dimethyl-N-(4-(9-phenyl-9H-carbazol-3-yl)phenyl)-9H-fluoren-2-amine and/or NPNPB. The HIL 540 may be omitted in compliance with a structure of the OLED 400.

Each of the HTL1 550 and the HTL2 650 may independently include, but is not limited to, TPD, DNTPD, NBP(NPD), CBP, poly-TPD, TFB, TAPC, DCDPA, N-(biphenyl-4-yl)-9,9-dimethyl-N-(4-(9-phenyl-9H-carbazol-3-yl)phenyl)-9H-fluoren-2-amine, N-(biphenyl-4-yl)-N-(4-(9-phenyl-9H-carbazol-3-yl)phenyl)biphenyl-4-amine and/or N-([1,1'-Biphenyl]-4-yl)-9,9-dimethyl-N-(4-(9-phenl-9H-carbazol-3-yl)phenyl)-9H-fluore n-2-amine. Each of the HIL 540, the HTL1 550 and the HTL2 650 may have a thickness, but is not limited to, between about 5 nm to about 200 nm, and preferably about 5 nm to about 100 nm.

Each of the ETL1 570 and ETL2 670 facilitates electron transportations in the first emitting unit 530 and the second emitting unit 630, respectively. Each of the ETL1 570 and the ETL2 670 may independently include, but is not limited to, oxadiazole-based compounds, triazole-based compounds, phenanthroline-based compounds, benzoxazole-based compounds, benzothiazole-based compounds, benzimidazole-based compounds, triazine-based compounds, and the like, respectively. As an example, each of the ETL1 570 and ETL2 670 may independently include, but is not limited to, Alq₃, PBD, spiro-PBD, Liq, TPBi, BAlq, Bphen, NBphen, BCP, TAZ, NTAZ, TpPyPB, TmPPPyTz, PFNBr, TPQ, TSPO1, ZADN and combination thereof, respectively.

The EIL 680 is disposed between the second electrode 420 and the ETL2 670, and can improve physical properties of the second electrode 420 and therefore, can enhance the lifetime of the OLED 400. In one exemplary aspect, the EIL 680 may include, but is not limited to, an alkali metal halide and/or alkaline earth metal halide such as LiF, CsF, NaF, BaF ₂ and the like, and/or an organic metal compound such as lithium benzoate, sodium stearate, and the like. Each of the ETL1 570, the ETL2 670 and the EIL 680 may have a thickness, but is not limited to, between about 10 nm to about 200 nm, preferably about 10 nm to about 100 nm.

Each of the EBL1 555 and the EBL2 655 may independently include, but is not limited to, TCTA, Tris[4-(diethylamino)phenyl]amine, N-(biphenyl-4-yl)-9,9-dimethyl-N-(4-(9-phenyl-9H-carbazol-3-yl)phenyl)-9H-fluorene-2 -amine, TAPC, MTDATA, mCP, mCBP, CuPc, DNTPD, TDAPB, DCDPA and/o 2,8-bis(9-phenyl-9H-carbazol-3-yl)dibenzo[b,d]thiophene, respectively.

Each of the HBL1 575 and the HBL2 675 may independently include, but is not limited to, oxadiazole-based compounds, triazole-based compounds, phenanthroline-based compounds, benzoxazole-based compounds, benzothiazole-based compounds, benzimidazole-based compounds, and triazine-based compounds. As an example, each of the HBL1 575 and the HBL2 675 may independently include, but is not limited to, BCP, BAlq, Alq ₃, PBD, spiro-PBD, Liq, B3PYMPM, DPEPO, 9-(6-(9H-carbazol-9-yl)pyridine-3-yl)-9H-3,9'-bicarbazole, TSPO1 and combination thereof, respectively.

The CGL 590 is disposed between the first emitting unit 530 and the second emitting unit 630. The CGL 590 includes an N-type CGL (N-CGL) 610 disposed adjacently to the first emitting unit 530 and a P-type CGL (P-CGL) 620 disposed adjacently to the second emitting unit 630. The N-type CGL 610 injects electrons into the first emitting unit 530 and the P-type CGL 620 injects holes into the second emitting unit 630.

As an example, the N-CGL 610 may be an organic layer doped with an alkali metal such as Li, Na, K and/or Cs and/or an alkaline earth metal such as Mg, Sr, Ba and/or Ra. For example, a host used in the N-CGL 610 may include, but is not limited to, an organic compound such as Bphen or MTDATA. The alkali metal or the alkaline earth metal may be doped by about 0.01 wt% to about 30 wt% in the N-type CGL 610.

The P-CGL 620 may include, but is not limited to, an inorganic material selected from the group consisting of tungsten oxide (WO ₓ), molybdenum oxide (MoO ₓ), beryllium oxide (Be ₂O ₃), vanadium oxide (V ₂O ₅) and combination thereof, and/or an organic material selected from the group consisting of NPD, HAT-CN, 2,3,5,6-Tetrafluoro-7,7,8,8-tetracyanoquinodimethane (F4TCNQ), TPD, N,N,N',N'-Tetranaphthalenyl-benzidine (TNB), TCTA, N,N'-dioctyl-3,4,9,10-perylenedicarboximide (PTCDI-C8) and combination thereof.

The EML2 660 may comprise a first host including the organic compound having the structure of Chemical Formulae 1 to 5, and a first dopant as green dopant. As an example, the first dopant may include the metal complex having the structure of Chemical Formula 6A or Chemical Formula 6B. The first dopant may comprise, but is not limited to, [Bis(2-phenylpyridine)](pyridyl-2-benzofuro[2,3-b]pyridine)iridium, fac-Ir(ppy) ₃, Ir(ppy) ₂(acac), Ir(mppy) ₃, Ir(npy) ₂acac, Ir(mppy) ₃, TEG, and the like.

If necessary, the EML2 660 may further comprise a seocnd host which can be used with the first host. The seocnd host may comprise, but is not limited to, BCzPh, CBP, TCP, TCTA, CDBP, DMFL-CBP, Spiro-CBP, DPEPO, PCzB-2C), mCzB-2CN, TCz1, and the like. When the EML2 660 includes the first host having the structure of Chemcial Formulae 1 to 5 and the second host as green host, the first host and the second host may be mixed, but is not limited to, with a weigh ratio between about 8:2 to about 2:8.

The contens of the first dopant in the EML2 660 may be between about 1 wt% to about 50 wt%, preferably about 1 wt% to about 30 wt%. For example, the EML2 660 may have a thickness, but is not limited to, between about 10 nm to about 200 nm, preferably about 20 nm to about 100 nm, and more preferably about 20 nm to about 50 nm.

The EML1 560 may be blue and/or red emiting material layers. In one exemplary aspect, the EML1 560 comprises the blue emitting material layer and the red emitting material layer. When the EML1 560 is the blue emitting material layer, the EML1 560 may emit blue color, sky blue color and/or deep blue colors.

As an example, the EML1 560 may comprise a lower emitting material layer (not shown) disposed between the EBL1 555 and the HBL1 575 and an upper emitting material layer (not shown) disposed between the lower emitting material layer and the HBL1 575. In this case, one of the lower emititng material layer (not shown) and the upper emititng material layer (not swhon) is the red emitting material layer, and the other of the lower emitting material layer and the upper emitting material layer is the blue emititng material layer.

As an example, the lower emitting material layer may comprise a third host as the red host and a second dopant as the red dopant. The upper emitting material layer may comprise a fourth host as a blue host and a third dopant as a blue dopant.

For example, the third host as the red host may comprise, but is not limited to, Bepp _{2,} Bepq ₂ and TBP3 as well as the second host described above. The second dopant as the red dopant may comprise the metal complex having the structure of Chemical Formula 7A or Chemical Formula 7B. As an example, the second dopant as the red dopant may comprise, but is not limited to, [Bis(2-(4,6-dimethyl)phenylquinoline)](2,2,6,6-tetramethylheptane-3,5-dionate)iridiu m(III), Hex-Ir(phq) ₂(acac), Hex-Ir(phq) ₃, Ir(Mphq) ₃, Ir(dpm)PQ ₂, Ir(dpm)(piq) ₂, Hex-Ir(piq) ₂(acac), Hex-Ir(piq) ₃, Ir(dmpq) ₃, Ir(dmpq) ₂(acac), Ir(mphmq) ₂(acac), and the like.

The fourth host as the blue host may comprise, but is not limited to, mCP, 9-(3-(9H-carbazol-9-yl)phenyl)-9H-carbazole-3-carbonitrile (mCP-CN), mCBP, CBP-CN, 9-(3-(9H-Carbazol-9-yl)phenyl)-3-(diphenylphosphoryl)-9H-carbazole (mCPPO1), 3,5-Di(9H-carbazol-9-yl)biphenyl (Ph-mCP), TSPO1, 9-(3'-9H-carbazol-9-yl)-[1,1'-biphenyl]-3-yl)-9H-pyrido[2,3-b]indole (CzBPCb), Bis(2-methylphenyl)diphenylsilane (UGH-1), 1,4-Bis(triphenylsilyl)benzene (UGH-2), 1,3-Bis(triphenylsilyl)benzene (UGH-3), 9,9-Spiorobifluoren-2-yl-diphenyl-phosphine oxide (SPPO1), 9,9'-(5-(Triphenylsilyl)-1,3-phenylene)bis(9H-carbazole) (SimCP), and the like.

[50] The third dopant as the blue dopant may comprise, but is not limited to, perylene, 4,4'-Bis[4-(di-p-tolylamino)styryl]biphenyl (DPAVBi), 4-(Di-p-tolylamino)-4-4'-[(di-p-tolylamino)styryl]stilbene (DPAVB), 4,4'-Bis[4-(diphenylamino)styryl]biphenyl (BDAVBi), 2,5,8,11-Tetra-tetr-butylperylene (TBPe), Bepp2, 9-(9-Phenylcarbazole-3-yl)-10-(naphthalene-1-yl)anthracene (PCAN), mer-Tris(1-phenyl-3-methylimidazolin-2-ylidene-C,C(2)'iridium(III) (mer-Ir(pmi) ₃), fac-Tris(1,3-diphenyl-benzimidazolin-2-ylidene-C,C(2)'iridium(III) (fac-Ir(dpbic) ₃), Bis(3,4,5-trifluoro-2-(2-pyridyl)phenyl-(2-carboxypyridyl)iridium(III) (Ir(tfpd) ₂pic), Tris(2-(4,6-difluoromethyl)pyridine)irdium((III) (Ir(Fppy) ₃), Bis[2-(4,6-difluorophenyl)pyridinato-C ²,N](picolinato)iridium(III) (FIrpic), and the like.

For example, each of the contents of the seocnd dopant and the third dopant in the red and blue emitting material layers may be between about 1 wt% to about 50 wt%, preferably about 1 wt% to about 30 wt%, respectively. Each of the upper and lower emitting material layers may have a thickness, but is not limited to, between about 10 nm to about 100 nm, preferably about 10 nm to about 50 nm.

In the above third aspect, the OLED 400 where the emitting materil layer incluidng the organic compound having the strucutre of Chemical Formulae 1 to 5, for example the EML2 660 is the green emitting material layer is expained. Alternatively, the emitting material layer incluidng the organic compound having the structure of Chemical Formulae 1 to 5 may emit yellow red green color or yellow green color.

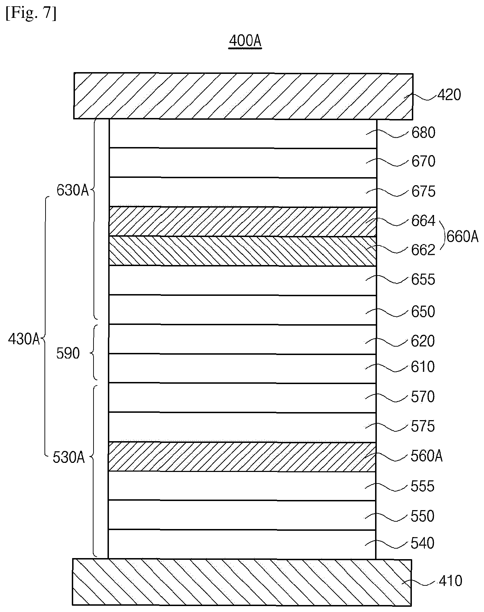

FIG. 7 is a schematic cross-sectional view illustrating an OLED having two emitting units in accordance with a fourth aspect of the present disclosure. As illustrated in FIG. 7, the OLED 400A comprise first and second electrodes 410 and 420 facing each other and an organic emissive layer 430A disposed between the first and second electrodes 410 and 420. The organic emissive layer 430A comprises a first emitting unit 530A disposed between the first electrode 410 and 420, a second emitting unit 630A disposed between the first emitting unit 530A and the second electrode 420, and a charge generation layer (CGL) 590 disposed between the first and second emitting units 530 and 630.

The first emitting unit 530A comprises the HIL 540, the HTL1 550, a first EML (EML1) 560A and the ETL1 570, and optionally comprises the EBL1 555 and/or the HBL2 575. The second emitting unit 630A comprises the HTL2 650, a second EML 660A, the ETL2 670 and the EIL 680, and optionally comprises the EBL2 655 and/or the HBL2 675.

The CGL 590 disposed between the first and second emitting units 530A and 630A comprises the N-type CGL 610 disposed adjacently to the first emitting unit 530A and the P-type CGL 620 disposed adjacently to the second emitting unit 630A. Each of the first and second electrodes 410 and 420, the CGL 590, the first emitting 630A unit except the EML1 560A and the second emitting unit 630A except the EML2 660A is identical to the corresponding elements in FIG. 6.

In this aspect, one of the EML1 560A and the EML2 660A may comprise the organic compound having the structure of Chemical Formulae 1 to 5 to emit red green or yellow green color. The other of the EML1 560A and the EML2 660A may emit blue color. Hereinafter, the OLED 400A where the EML2 660A comprises the organic compound having the structure of Chemical Formulae 1 to 5 will be explained.

The EML2 660A comprises a lower EML 662 disposed between the EBL2 655 and the HBL2 655 and an upper EML 664 disposed between the lower EML 662 and the HBL2 675. One of the lower EML 662 and the upper EML 664 may comprise any organic compound having the structure of Chemical Formulae 1 to 5 to emit green color, and the other of the lower EML 662 and the upper EML 664 may emit red or yellow color. Hereinafter, the EML2 660A where the lower EML 662 emits green color and the upper EML 664 emits red or yellow color will be explained.

The lower EML 662 of the EML2 660A may comprise the first host and the first dopant, and optionally may further comprise the second host in addition to the first host. The first host may comprise the organic compound having the structure of Chemical Formulae 1 to 5. The first dopant may be the green dopant. The first dopant may comprise the metal complex having the structure of Chemical Formula 6A or Chemical Formula 6B. As an example, the first dopant may comprise, but is not limited to, [Bis(2-phenylpyridine)](pyridyl-2-benzofuro[2,3-b]pyridine)iridium, fac-Ir(ppy) ₃, Ir(ppy) ₂(acac), Ir(mppy) ₃, Ir(npy) ₂acac, Ir(mppy) ₃, TEG, and the like.

The second host may be the green host and may comprise, but is not limited to, BCzPh, CBP, TCP, TCTA, CDBP, DMFL-CBP, Spiro-CBP, DPEPO, PCzB-2C), mCzB-2CN, TCz1, and the like.

The upper EML 664 of the EML2 660A may comprise the third host and the second dopant. In one exemplary aspect, the third host may be the red host and the second dopant may be the red dopant. The third host as the red host may comprise, but is not limited to, Bepp ₂, Bepq ₂ and TBP3 as well as the second host described above. The second dopant as the red dopant may comprise the metal complex having the structure of Chemical Formula 7A or Chemical Formula 7B. As an example, the second dopant as the red dopant may comprise, but is not limited to, [Bis(2-(4,6-dimethyl)phenylquinoline)](2,2,6,6-tetramethylheptane-3,5-dionate)iridiu m(III), Hex-Ir(phq) ₂(acac), Hex-Ir(phq) ₃, Ir(Mphq) ₃, Ir(dpm)PQ ₂, Ir(dpm)(piq) ₂, Hex-Ir(piq) ₂(acac), Hex-Ir(piq) ₃, Ir(dmpq) ₃, Ir(dmpq) ₂(acac), Ir(mphmq) ₂(acac), and the like.

In another exemplary aspect, the third host in the upper EML 664 may be the yellow host and the second dopant may be the yellow dopant. The third host as the yellow host may be the same as the red host. The second dopant as the yellow dopant may comprise, but is not limited to, Rubrene, TBRb, Ir(BT) ₂(acac), Ir(fbi) ₂(acac), fac-Ir(ppy) ₂Pc, FPQIrpic, and the like.

For example, when the lower and upper EMLs 662 and 664 includes the dopant, each of the contents of the dopant in each of the lower and upper EMLs 662 and 664 may be between about 1 wt% to about 50 wt%, preferably about 1 wt% to about 30 wt%, respectively. Each of the lower EML 662 and the upper EML 664 may have a thickness, but is not limited to, between about 10 nm to about 100 nm, preferably about 10 nm to about 50 nm.

The EML1 560A may be blue emitting material layer. The EML1 560A may emit blue color, sky blue color and/or deep blue colors. The EML1 560A may comprise the fourth host as the blue host and the third dopant as the blue dopant. For example, the fourth host as the blue host may comprise, but is not limited to, mCP, mCP-CN, mCBP, CBP-CN, mCPPO1, Ph-mCP, TSPO1, CzBPCb, UGH-1), UGH-2, UGH-3, SPPO1), SimCP, and the like. The third dopant as the blue dopant may comprise, but is not limited to, perylene, DPAVBi, DPAVB, BDAVBi, TBPe, Bepp2, PCAN, mer-Ir(pmi) ₃, ac-Ir(dpbic) ₃, Ir(tfpd) ₂pic, Ir(Fppy) ₃, FIrpic, and the like.

The contents of the thrid dopant as the blue dopant in the EML1 560A may be between about 1 wt% to about 50 wt%, preferably about 1 wt% to about 30 wt%. For example, the EML1 560A may have a thickness, but is not limited to, between about 10 to 200 nm, preerably about 20 nm to about 100 nm, and more preferably about 20 nm to about 50 nm.

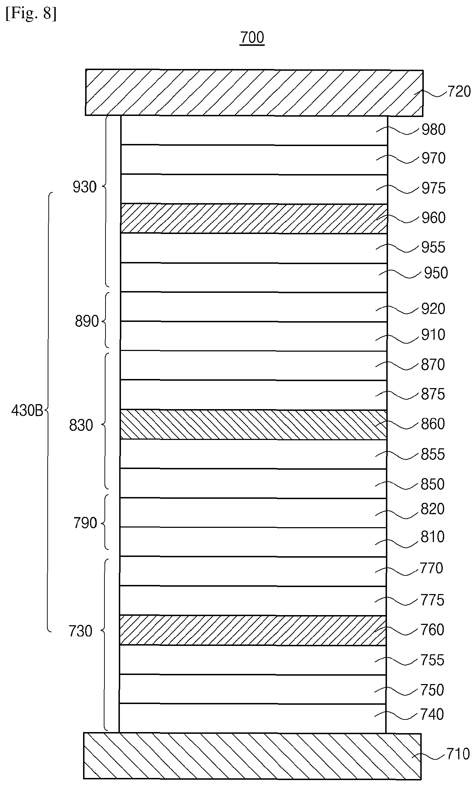

Alternatively, an OLED may have a tandem structure in which three or more emitting units are laminated. FIG. 8 is a schematic cross-sectional view illustrating an OLED having three emitting units in accordance with a fifth aspect of the present disclosure.

As illustrated in FIG. 8, the OLED 700 comprise first and second electrodes 710 and 720 facing each other and an organic emissive layer 430B disposed between the first and second electrodes 710 and 720. The organic emissive layer 430B comprises a first emitting unit 730 disposed between the first and second electrodes 710 and 720, a second emitting unit 830 disposed between the first emitting unit 730 and the second electrode 720, a third emitting unit 930 disposed between the second emitting unit 830 and the second electrode 720, a first CGL (CGL1) 790 disposed between the first and second emitting units 730 and 830 and a second CGL (CGL2) 890 disposed between the second and third emitting units 830 and 930.

The first electrode 710 may be an anode and include a conductive material having a relatively large work function values, for example, TCO such as ITO, IZO, SnO, ZnO, ICO, AZO, and the like. The second electrode 720 may be a cathode and include a conductive material having a relatively small work function values such as Al, Mg, Ca, Ag, alloy thereof or combination thereof. As an example, each of the first and second electrodes 710 and 720 may be laminated with a thickness of, but is not limited to, about 30 nm to about 300 nm.

The first emitting unit 730 comprises a HIL 740, a first HTL (HTL1) 750, a first EML (EML1) 760 and a first ETL (ETL1) 770 each of which is disposed sequentially on the first electrode 710. Alternatively, the first emitting unit 730 may further comprise a first EBL (EBL1) 755 disposed between the HTL1 750 and the EML1 760 and/or a first HBL (HBL1) 775 disposed between the EML1 760 and the ETL1 770.

The second emitting unit 830 comprises a second HTL (HTL2) 850, a second EML (EML2) 860 and a second ETL (ETL2) 870 each of which is disposed sequentially on the CGL1 790. Alternatively, the second emitting unit 830 may further comprise a second EBL (EBL2) 855 disposed between the HTL2 850 and the EML2 860 and/or a second HBL (HBL2) 875 disposed between the EML2 860 and the ETL2 870.

The third emitting unit 930 comprises a third HTL (HTL3) 950, a third EML (EML3) 960, a third ETL (ETL3) 970 and an EIL 980 each of which is disposed sequentially on the CGL2 890. Alternatively, the third emitting unit 930 may further comprise a third EBL (EBL3) 955 disposed between the HTL3 950 and the EML3 960 and/or a third HBL (HBL3) 975 disposed between the EML3 960 and the ETL3 970.

At least one of the EML1 760, the EML2 860 and the EML3 960 may comprise the organic compound having the structure of Chemical Formulae 1 to 5 to emit green color. For example, one of the EML1 760, the EML2 860 and the EML3 960 may emit green color, another of the EML1 760, the EML2 860 and the EML3 960 may emit red color, and the third of the EML1 760, the EML2 860 and the EML3 960 may emit blue color so that the OLED 700 can implement white color. Hereinafter, the OLED 700 where the EML1 860 includes the organic compound having the structure of Chemical Formulae 1 to 5 to emit green color, the EML1 760 emits red color and the EML3 960 emits blue color will be explained.

The HIL 740 is disposed between the first electrode 710 and the HTL1 750 and improves an interface property between the inorganic first electrode 710 and the organic HTL1 750. In one exemplary embodiment, the HIL 740 include, but is not limited to, MTDATA, NATA, 1T-NATA, 2T-NATA, CuPc, TCTA, NPB(NPD), HAT-CN, TDAPB, PEDOT/PSS, N-(biphenyl-4-yl)-9,9-dimethyl-N-(4-(9-phenyl-9H-carbazol-3-yl)phenyl)-9H-fluoren-2-amine and/or NPNPB. The HIL 740 may be omitted in compliance with the structure of the OLED 700.

Each of the HTL1 750, the HTL2 850 and the HTL3 950 may independently include, but is not limited to, TPD, DNTPD, NBP(NPD), CBP, poly-TPD, TFB, TAPC, DCDPA, N-(biphenyl-4-yl)-9,9-dimethyl-N-(4-(9-phenyl-9H-carbazol-3-yl)phenyl)-9H-fluoren-2-amine, N-(biphenyl-4-yl)-N-(4-(9-phenyl-9H-carbazol-3-yl)phenyl)biphenyl-4-amine and/or N-([1,1'-Biphenyl]-4-yl)-9,9-dimethyl-N-(4-(9-phenl-9H-carbazol-3-yl)phenyl)-9H-fluore n-2-amine. Each of the HIL 740, the HTL1 750, the HTL2 850 and the HTL3 950 may have a thickness, but is not limited to, between about 5 nm to about 200 nm, and preferably about 5 nm to about 100 nm.

Each of the ETL1 770, the ETL2 870 and the ETL3 970 facilitates electron transportations in each of first to third emitting units 730, 830 and 930, respectively. Each of the ETL1 770, the ETL2 870 and the ETL3 970 may independently include, but is not limited to, oxadiazole-based compounds, triazole-based compounds, phenanthroline-based compounds, benzoxazole-based compounds, benzothiazole-based compounds, benzimidazole-based compounds, triazine-based compounds, and the like, respectively. As an example, each of the ETL1 770, the ETL2 870 and the ETL3 970 may independently include, but is not limited to, Alq ₃, PBD, spiro-PBD, Liq, TPBi, BAlq, Bphen, NBphen, BCP, TAZ, NTAZ, TpPyPB, TmPPPyTz, PFNBr, TPQ, TSPO1, ZADN and combination thereof, respectively.

The EIL 980 is disposed between the second electrode 720 and the ETL3 970, and can improve physical properties of the second electrode 720 and therefore, can enhance the lifetime of the OLED 700. In one exemplary aspect, the EIL 980 may include, but is not limited to, an alkali metal halide and/or alkaline earth halide such as LiF, CsF, NaF, BaF ₂ and the like, and/or an organic metal compound such as lithium benzoate, sodium stearate, and the like. Each of the ETL1 770, the ETL2 870, the ETL3 970 and the EIL 980 may have a thickness, but is not limited to, between about 10 nm to about 200 nm, preferably about 10 nm to about 100 nm.

Each of the EBL1 755, the EBL2 855 and the EBL3 955 may independently include, but is not limited to, TCTA, Tris[4-(diethylamino)phenyl]amine, N-(biphenyl-4-yl)-9,9-dimethyl-N-(4-(9-phenyl-9H-carbazol-3-yl)phenyl)-9H-fluorene-2 -amine, TAPC, MTDATA, mCP, mCBP, CuPc, DNTPD, TDAPB, DCDPA and/o 2,8-bis(9-phenyl-9H-carbazol-3-yl)dibenzo[b,d]thiophene, respectively.

Each of the HBL1 775, the HBL2 875 and the HBL3 975 may independently include, but is not limited to, oxadiazole-based compounds, triazole-based compounds, phenanthroline-based compounds, benzoxazole-based compounds, benzothiazole-based compounds, benzimidazole-based compounds, and triazine-based compounds. As an example, each of the HBL1 775, the HBL2 875 and the HBL3 975 may independently include, but is not limited to, BCP, BAlq, Alq ₃, PBD, spiro-PBD, Liq, B3PYMPM, DPEPO, 9-(6-(9H-carbazol-9-yl)pyridine-3-yl)-9H-3,9'-bicarbazole, TSPO1 and combination thereof, respectively.

The CGL1 790 is disposed between the first emitting unit 730 and the second emitting unit 830 and the CGL2 890 is disposed between the second emitting unit 830 and the third emitting unit 930. The CGL1 790 includes a first N-type CGL (N-CGL1) 710 disposed adjacently to the first emitting unit 730 and a first P-type CGL (P-CGL1) 820 disposed adjacently to the second emitting unit 830. The CGL2 890 includes a second N-type CGL (N-CGL2) 910 disposed adjacently to the second emitting unit 830 and a second P-type CGL (P-CGL2) 920 disposed adjacently to the third emitting unit 930. Each of the N-CGL1 810 and the N-CGL2 910 injects electrons into each of the first emitting unit 730 and the second emitting unit 830, respectively, and each of the P-CGL1 820 and the P-CGL2 920 injects holes into each of the second emitting unit 830 and the third emitting unit 930, respectively.

As an example, each of the N-CGL1 810 and the N-CGL2 910 may be an organic layer doped with an alkali metal such as Li, Na, K and/or Cs and/or an alkaline earth metal such as Mg, Sr, Ba and/or Ra. For example, a host used in each of the N-CGL1 810 and the N-CGL2 910 may include, but is not limited to, an organic compound such as Bphen or MTDATA. The alkali metal or the alkaline earth metal may be doped by about 0.01 wt% to about 30 wt% in each of the N-CGL1 810 and the N-CGL2 910.

Each of the P-CGL1 820 and the P-CGL2 920 may include, but is not limited to, an inorganic material selected from the group consisting of tungsten oxide (WO ₓ), molybdenum oxide (MoO ₓ), beryllium oxide (Be ₂O ₃), vanadium oxide (V ₂O ₅) and combination thereof, and/or an organic material selected from the group consisting of NPD, HAT-CN, F4TCNQ, TPD, N,N,N',N'-Tetranaphthalenyl-benzidine (TNB), TCTA, N,N'-dioctyl-3,4,9,10-perylenedicarboximide (PTCDI-C8) and combination thereof.

In this aspect, the EML2 860 may comprise the first host having the structure of Chemical Formula 1 and the first dopant as the green dopant, and optionally may further comprise the second host as the green host in addition to the first host.

As an example, the second host may comprise, but is not limited to, BCzPh, CBP, TCP, TCTA, CDBP, DMFL-CBP, Spiro-CBP, DPEPO, PCzB-2C), mCzB-2CN, TCz1, and the like. The first dopant may comprise, but is not limited to, [Bis(2-phenylpyridine)](pyridyl-2-benzofuro[2,3-b]pyridine)iridium, fac-Ir(ppy) ₃, Ir(ppy) ₂(acac), Ir(mppy) ₃, Ir(npy) ₂acac, Ir(mppy) ₃, TEG, and the like.

The contens of the first dopant in the EML2 860 may be between about 1 wt% to about 50 wt%, preferably about 1 wt% to about 30 wt%. For example, the EML2 860 may have a thickness, but is not limited to, between about 10 nm to about 200 nm, preferably about 20 nm to about 100 nm, and more preferably about 20 nm to about 50 nm.

The EML1 760 may be the red emitting material layer. In this case, the EML1 760 may comprise the third host as the red host and the second dopant as the red dopant. The third host as the red host in the EML1 760 may comprise, but is not limited to, Bepp ₂, Bebq ₂, TPB3 in addition to the second host described above.

The seocnd dopant as the red dopant may comprise the metal complex having the structure of Chemical Formula 7A or Chemical Formula 7B. As an example, the seocnd dopant as the red dopant may comprise, but is not limited to, [Bis(2-(4,6-dimethyl)phenylquinoline)](2,2,6,6-tetramethylheptane-3,5-dionate)iridiu m(III), Hex-Ir(phq) ₂(acac), Hex-Ir(phq) ₃, Ir(Mphq) ₃, Ir(dpm)PQ ₂, Ir(dpm)(piq) ₂, Hex-Ir(piq) ₂(acac), Hex-Ir(piq) ₃, Ir(dmpq) ₃, Ir(dmpq) ₂(acac), Ir(mphmq) ₂(acac), and the like.

The EML3 969 may comprise the fourth host as the blue host and the third dopant as the blue dopant. The fourth host as the blue host in the EML3 860 may comprise, but is not limited to, , mCP, mCP-CN, mCBP, CBP-CN, mCPPO1, Ph-mCP, TSPO1, CzBPCb, UGH-1), UGH-2, UGH-3, SPPO1), SimCP, and the like. The third dopant as the blue dopant may comprise, but is not limited to, perylene, DPAVBi, DPAVB, BDAVBi, TBPe, Bepp2, PCAN, mer-Ir(pmi) ₃, ac-Ir(dpbic) ₃, Ir(tfpd) ₂pic, Ir(Fppy) ₃, FIrpic, and the like.

For example, each of the contents of the first to third dopant in each of the EML1 760, the EML2 860 and the EML3 960 may be between about 1 wt% to about 50 wt%, preferably about 1 wt% to about 30 wt%, respectively. Each of the EML1 760, the EML2 860 and the EML3 960 may have a thickness, but is not limited to, between about 10 nm to about 100 nm, preferably about 10 nm to about 50 nm.

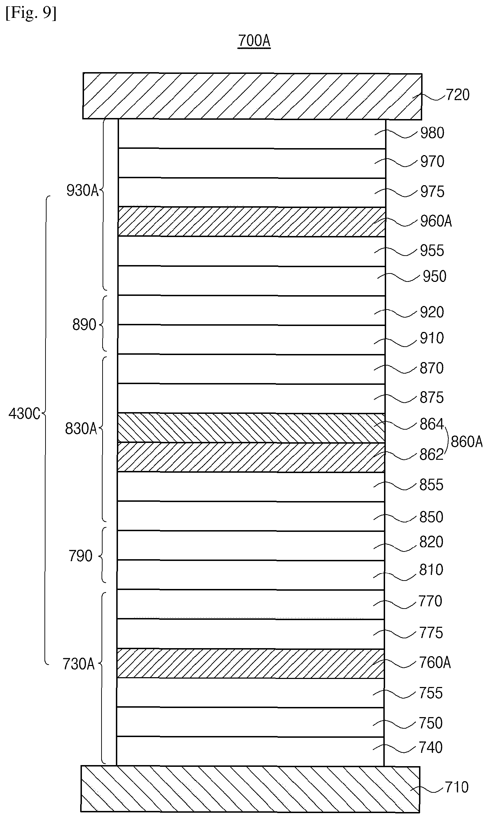

In the above fifth aspect, the OLED 700 where the emittng materil layer incluidng the organic compound having the strucutre of Chemical Formulae 1 to 5, for example the EML2 860 is the green emitting material layer is expained. Alternatively, the emitting material layer incluidng the organic compound having the structure of Chemical Formulae 1 to 5 may emit yellow red green color or yellow green color. FIG. 9 is a schematic cross-sectional view illustrating an OLED having three emitting units in accordance with a sixth aspect of the present disclosure.

As illustrated in FIG. 9, the OLED 700A comprise first and second electrodes 710 and 720 facing each other and an organic emissive layer 430C disposed between the first and second electrodes 710 and 720. The organic emissive layer 430C comprises a first emitting unit 730A disposed between the first and second electrodes 710 and 720, a second emitting unit 830A disposed between the first emitting unit 730A and the second electrode 720, a third emitting unit 930A disposed between the second emitting unit 830A and the second electrode 720, a CGL1 790 disposed between the first and second emitting units 730A and 830A, and a CGL2 890 disposed between the second and third emitting units 830A and 930A..

The first emitting unit 730A comprises the HIL 740, the HTL1 750, an EML1 760A, the ETL1 770, and optionally comprises the EBL1 755 and/or the HBL1 775. The second emitting unit 830A comprises the HTL2 850, an EML2 860A, the ETL2 870, and optionally the EBL2 855 and/or the HBL2 875. The third emitting unit 930A comprises the HTL3 950, an EML3 960A, the ETL3 970 and the EIL 980, and optionally the EBL3 955 and/or the HBL3 975.

The CGL1 790 comprises the N-CGL1 810 disposed adjacently to the first emitting unit 730A and the P-CGL1 820 disposed adjacently to the second emitting unit 830A. The CGL2 890 comprises the N-CGL2 910 disposed adjacently to the second emitting unit 830A and the P-CGL2 920 disposed adjacently to the third emitting unit 930A. Each of the first and second electrodes 710 and 720, the CGL1 790, the CGL2 890, the first emitting 730A unit except the EML1 760A, the second emitting unit 830A except the EML2 860A, the third emitting unit 930A except the EML3 960A is identical to the corresponding elements in FIG. 8.

In this aspect, one of the EML1 760A, EML2 860A and the EML3 960A may comprise the organic compound having the structure of Chemical Formulae 1 to 5. Hereinafter, the OLED 700A where the EML2 860A comprise the organic compound will be explained.

The EML2 860A comprises a lower EML 862 disposed between the EBL2 855 and the HBL2 855 and an upper EML 864 disposed between the lower EML 862 and the HBL2 875. One of the lower EML 862 and the upper EML 864 may comprise any organic compound having the structure of Chemical Formulae 1 to 5 to emit green color, and the other of the lower EML 862 and the upper EML 864 may emit red or yellow color. Hereinafter, the EML2 860A where the lower EML 862 emits green color and the upper EML 864 emits red or yellow color will be explained.

The lower EML 862 of the EML2 860A may comprise the first host and the first dopant, and optionally may comprise the second host which can be used with the first host. The first host may comprise the organic compound having the structure of Chemical Formulae 1 to 5. The first dopant may be the green dopant and may comprise the metal complex having the structure of Chemical Formula 6A or Chemical Formula 6B. As an example, the first dopant may comprise, but is not limited to, [Bis(2-phenylpyridine)](pyridyl-2-benzofuro[2,3-b]pyridine)iridium, fac-Ir(ppy) ₃, Ir(ppy) ₂(acac), Ir(mppy) ₃, Ir(npy) ₂acac, Ir(mppy) ₃, TEG, and the like.

The second host may be the green host and may comprise, but is not limited to, BCzPh, CBP, TCP, TCTA, CDBP, DMFL-CBP, Spiro-CBP, DPEPO, PCzB-2C), mCzB-2CN, TCz1, and the like.

The upper EML 864 of the EML2 860A may comprise the third host and the second dopant. In one exemplary aspect, the third host may be the red host and the second dopant may be the red dopant. The third host as the red host may comprise, but is not limited to, Bepp ₂, Bepq ₂ and TBP3 as well as the second host described above.

The second dopant as the red dopant may comprise the metal complex having the structure of Chemical Formula 7A or Chemical Formula 7B. As an example, the second dopant as the red dopant may comprise, but is not limited to, [Bis(2-(4,6-dimethyl)phenylquinoline)](2,2,6,6-tetramethylheptane-3,5-dionate)iridiu m(III), Hex-Ir(phq) ₂(acac), Hex-Ir(phq) ₃, Ir(Mphq) ₃, Ir(dpm)PQ ₂, Ir(dpm)(piq) ₂, Hex-Ir(piq) ₂(acac), Hex-Ir(piq) ₃, Ir(dmpq) ₃, Ir(dmpq) ₂(acac), Ir(mphmq) ₂(acac), and the like.

In another exemplary aspect, the third host in the upper EML 864 may be the yellow host and the second dopant may be the yellow dopant. The third host as the yellow host may be the same as the red host. The second dopant as the yellow dopant may comprise, but is not limited to, Rubrene, TBRb, Ir(BT) ₂(acac), Ir(fbi) ₂(acac), fac-Ir(ppy) ₂Pc, FPQIrpic, and the like.

For example, when the lower and upper EMLs 8662 and 864 includes the dopant, each of the contents of the dopant in each of the lower and upper EMLs 862 and 864 may be between about 1 wt% to about 50 wt%, preferably about 1 wt% to about 30 wt%, respectively. Each of the lower EML 862 and the upper EML 864 may have a thickness, but is not limited to, between about 10 nm to about 100 nm, preferably about 10 nm to about 50 nm.

Each of the EML1 760A and the EML3 960A may be blue emitting material layer, respectively. Each of the EML1 760A and the EML3 960 may emit blue color, sky blue color and/or deep blue colors, respectively. Each of the EML1 760A and the EML2 960A may independently comprise the fourth host as the blue host and the third dopant as the blue dopant. For example, the fourth host as the blue host may comprise, but is not limited to, mCP, mCP-CN, mCBP, CBP-CN, mCPPO1, Ph-mCP, TSPO1, CzBPCb, UGH-1), UGH-2, UGH-3, SPPO1), SimCP, and the like. The third dopant as the blue dopant may comprise, but is not limited to, perylene, DPAVBi, DPAVB, BDAVBi, TBPe, Bepp2, PCAN, mer-Ir(pmi) ₃, ac-Ir(dpbic) ₃, Ir(tfpd) ₂pic, Ir(Fppy) ₃, FIrpic, and the like.

The contents of the thrid dopant as the blue dopant in each of the EML1 760A and the EML3 960A may be between about 1 wt% to about 50 wt%, preferably about 1 wt% to about 30 wt%. For example, each of the EML1 760A and the EML3 960A may have a thickness, but is not limited to, between about 10 to 200 nm, preerably about 20 nm to about 100 nm, and more preferably about 20 nm to about 50 nm.

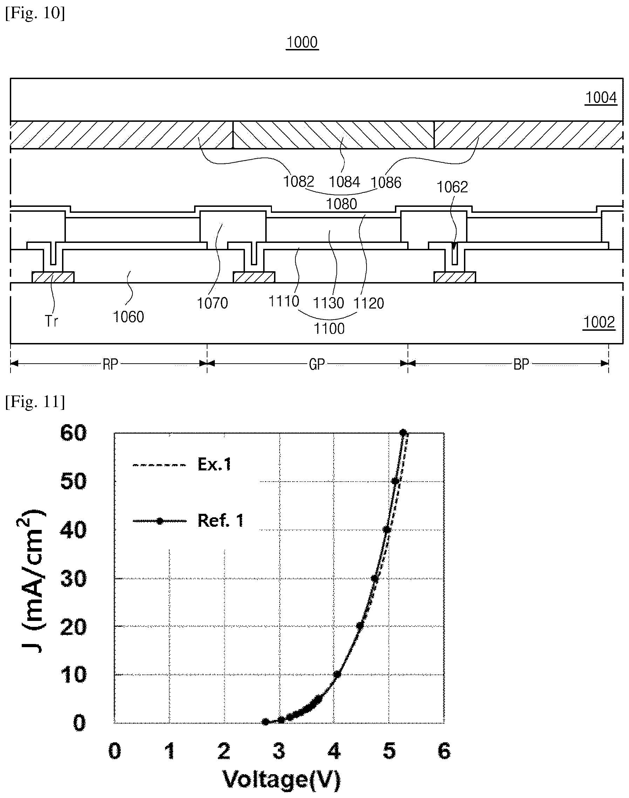

In another exemplary aspect, an organic light emitting device may comprise a color conversion film. FIG. 10 is a schematic cross-sectional view illustrating an organic light emitting display device in accordance with a third aspect of the present disclosure.

As illustrated in FIG. 10, the organic light emitting display device 1000 comprises a first substrate 1002 that defines each of a red pixel RP, a green pixel GP and a blue pixel BP, a second substrate 1004 facing the first substrate 1002, a thin film transistor Tr over the first substrate 1002, an OLED 1100 disposed between the first and second substrates 1002 and 1004 and emitting blue (B) light or white (W) light and a color conversion layer 1080 disposed between the OLED 1100 and the second substrate 1004. Although not shown in FIG. 10, a color filter may be formed disposed between the second substrate 1004 and the respective color conversion layer 1080.

The thin film transistor Tr is disposed over the first substrate 1002 correspondingly to each of the red pixel RP, the green pixel GP and the blue pixel BP. A passivation layer 1060, which has a drain contact hole 1062 exposing one electrode, for example a drain electrode (not shown), constituting the thin film transistor Tr, is formed with covering the thin film transistor Tr over the whole first substrate 1002.

The OLED 1100, which includes a first electrode 1110, an organic emissive layer 1130 and a second electrode 1120, is disposed over the passivation layer 1060. The first electrode 1110 may be connected to the drain electrode (not shown) of the thin film transistor Tr through the drain contact hole 1062. In addition, a bank layer 1070 covering edges of the first electrode 1110 is formed at the boundary between the red pixel RP, the green pixel GP and the blue pixel BP. In this case, the OLED 1100 may have a structure of FIGS. 6 to 9 and can emit b white color. The OLED 1100 is disposed in each of the red pixel RP, the green pixel GP and the blue pixel BP to provide white color.

The color conversion layer 1080 may include a first color conversion layer 1082 corresponding to the red pixel RP, a second color conversion layer 1084 corresponding to the green pixel GP and a third color conversion layer 1086 corresponding to the blue pixel BP. As an example, the color conversion layer 1080 may include an inorganic luminescent material such as quantum dot (QD).

The white color light emitted from the OLED 1100 in the red pixel RP is converted into red color light by the first color conversion layer 1082, the white color light emitted from the OLED 1100 in the green pixel GP is converted into green color light by the second color conversion layer 1084 and the white color light emitted from the OLED 1100 in the blue pixel BP is converted into blue color light by the third color conversion layer 1086.. Accordingly, the organic light emitting display device 1100 can implement a color image.

In addition, when the light emitted from the OLED 1100 is displayed through the first substrate 1002, the color conversion layer 1080 may be disposed between the OLED 1100 and the first substrate 1002.

### Examples

### Synthesis Example 1: Synthesis of Compound EH-2

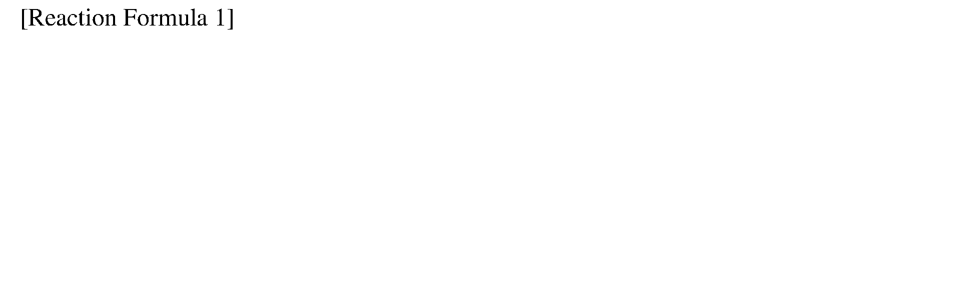

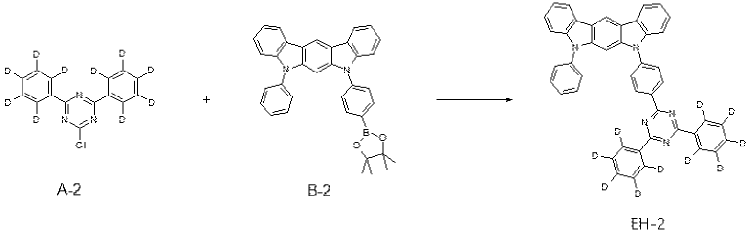

A-2 (3.0 g, 10.8 mmol), B-2 (6.6 g, 12.4 mmol), Pd(PPh ₃) ₄ (tetrakis(triphenylphosphine)palladium(II), 0.25 g, 0.22 mol), 2M K ₂CO ₃ aqueous solution (15 mL), toluene (150 mL) and THF (150 mL) were put into a 250 mL round-bottom flask under argon atmosphere, and then the solution was fluxed with stirring, After termination of reaction was confirmed with TLC (thin layer chromatography), an organic layer was separated from the reaction solution, was distilled under vacuum, and then was purified with a column chromatography to obtain Compound EH-2.

### Synthesis Example 2: Synthesis of Compound EH-7

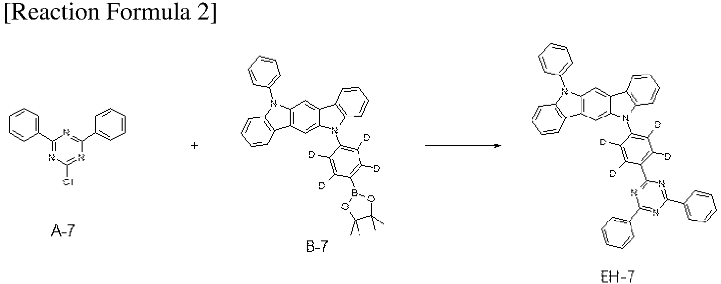

A-7 (0.8 g, 2.9 mmol), B-7 (1.2 g, 2.2 mmol), Pd(PPh ₃) ₄ (52 mg, 44.6 µmol), 2M K ₂CO ₃ aqueous solution (5 mL), toluene (20 mL) and THF (5 mL) were put into a 250 mL round-bottom flask under argon atmosphere, and then the solution was fluxed with stirring, After termination of reaction was confirmed with TLC, an organic layer was separated from the reaction solution, was distilled under vacuum, and then was purified with a column chromatography to obtain Compound EH-7.

### Synthesis Example 3: Synthesis of Compound EH-20

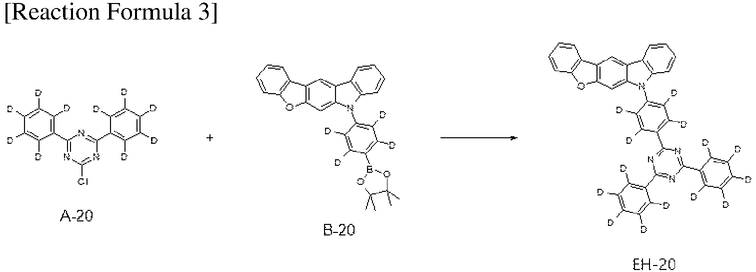

[1] A-20 (0.9 g, 3.4 mmol), B-20 (1.5 g, 3.2 mmol), Pd(PPh ₃) ₄ (75 mg, 64.7 µmol), 2M K ₂CO ₃ aqueous solution (7 mL), toluene (25 mL) and THF (5 mL) were put into a 250 mL round-bottom flask under argon atmosphere, and then the solution was fluxed with stirring, After termination of reaction was confirmed with TLC, an organic layer was separated from the reaction solution, was distilled under vacuum, and then was purified with a column chromatography to obtain Compound EH-20.

### Synthesis Example 4: Synthesis of Compound EH-49

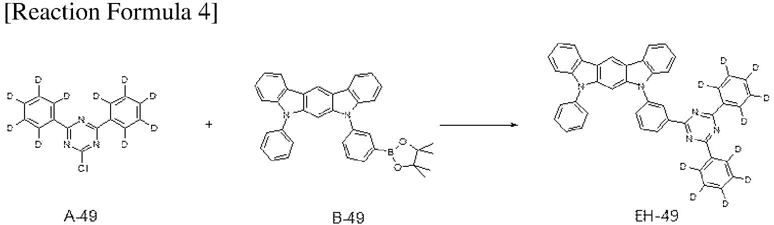

[1] A-49 (3.0 g, 10.8 mmol), B-49 (6.6 g, 12.4 mmol), Pd(PPh ₃) ₄ (0.25 g, 0.22 mol), 2M K ₂CO ₃ aqueous solution (15 mL), toluene (150 mL) and THF (60 mL) were put into a 250 mL round-bottom flask under argon atmosphere, and then the solution was fluxed with stirring, After termination of reaction was confirmed with TLC, an organic layer was separated from the reaction solution, was distilled under vacuum, and then was purified with a column chromatography to obtain Compound EH-49.

### Example 1 (Ex.1): Fabrication of OLED

[2] An OLED in which the Compound EH-2 is applied into an EML and includes single emitting unit implementing red/green colors was fabricated. An ITO (including reflective layer; 40 mm X 40 mm X 0.5 nm) attached glass substrate was ultrasonic washed with isopropyl alcohol, acetone and DI water for 5 minutes, and then dried in a oven at 100 °C. The substrate was treated with O ₂ plasma under vacuum for 2 minutes, and then was transferred to a vacuum deposition chamber in order to deposit other layers on the substrate. An organic layer was deposited by evaporation by a heated boat under 5 X 10 ⁻⁷ torr with a deposition rate 1 Å in the following order :

[3] An HIL (HAT-CN, 50 Å); an HTL (N-([1,1'-Biphenyl]-4-yl)-9,9-dimethyl-N-(4-(9-phenl-9H-carbazol-3-yl)phenyl)-9H-fl uoren-2-amine, 200 Å); lower EML (red host (Bepp2): red dopant (Bis(2-(4,6-dimethyl)phenylquinoline)(2,2,6,6-tetramethylheptane-3,5-dionate)iridium (III) = 97:3 by weight, 200 Å); upper EML (Host (first host (EH-2): second host (Bczph) = 50: 50 by weight): green dopant (([Bis(2-phenylpyridine)](pyridyl-2-benzofuro[2,3-b]pyridine)iridium) = 85: 15 by weight, 200 Å); an ETL (ZADN, 200 Å); an EIL (LiF, 10 Å); and cathode (Al, 100 Å).

[4] And then, cappling layer (CPL) was deposited over the cathode and the device was encapsualted by glass. After deposition of emissve layer and the cathode, the OLED was transferred from the depostion chamber to a dry box for film formation, followed by encapsulation using UV-curable epoxy resin and moisture getter.

### Examples 2 (Ex. 2): Fabrication of OLED

[5] An OLED in which the Compound EH-2 is applied into an EML and includes single emitting unit implementing green color was fabricated. The OLED was fabricated using the same material as Example 1, except that single green EML (Host (first host (EH-2): second host (Bczph) = 50: 50 by weight): green dopant (([Bis(2-phenylpyridine)](pyridyl-2-benzofuro[2,3-b]pyridine)iridium) = 85: 15 by weight, 200 Å) was used.

### Examples 3 (Ex. 3): Fabrication of OLED

[6] An OLED was fabricated using the same material as Example 1, except that Compound EH-20 was used as the first host in the upper EML instead of the Compound EH-2.

### Compartive Example 1 (Ref. 1): Fabrication of OLED

[7] An OLED was fabricated using the same material as Example 1, except that the following Compound A was used as the first host in the upper EML instead of the Compound EH-2

### Comparative Examples 2-4 (Ref. 2-4): Fabrication of OLED

[8] An OLED was fabricated using the same material as Example 2, except that each of the following Compound C (Ref. 2), Compound B (Ref. 3) and Compound A (Ref. 4) was used as the host in the EML instead of the Compound EH-2.

### Compartive Example 5 (Ref. 5): Fabrication of OLED

[9] An OLED was fabricated using the same material as Example 3, except that the following Compound D was used as the first host in the upper EML instead of the Compound EH-20

### [Reference Compound]

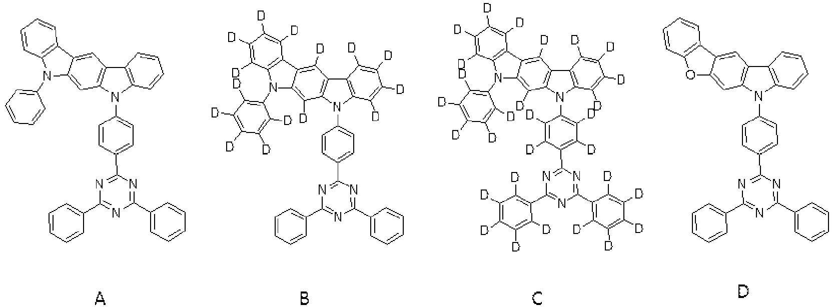

### Experimental Example 1: Measurement of Luminous Properties of OLED

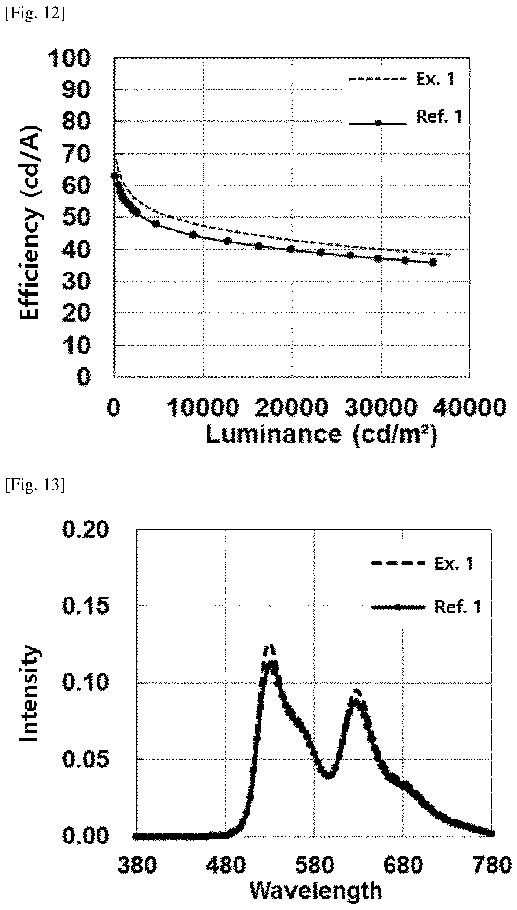

Each of the OLED fabricated by Ex. 1 and Ref. 1 was connected to an external power source and then luminous properties for all the diodes were evaluated using a constant current source (KEITHLEY) and a photometer PR650 at room temperature. In particular, driving voltage-current density, IVL (current-voltage-luminance) as well as driving voltage (V), current efficiency (cd/A), External Quantum Efficiency (EQE, %) and CIE color coordinate at 10 mA/cm ² current density were measured. In addition, luminance-current efficiency, intensity of electro-luminescence peak by wavelengths and lift time (LT) at 50 mA/ mA/cm ² current density were measured. The results thereof are shown in the following Table 1 and FIGS. 11-14.

**[Table 1]**

| Luminous Properties of OLED | | | | | | LT50 mA/cm ² |
|---|---|---|---|---|---|---|
| I-V-L 10 mA/cm ² | | | | | | |
| Sample | V | cd/A | EQE (%) | CIE(x) | CIE(y) | |
| Ex. 1 | 4.1 | 51.4 | 21.0 | 0.432 | 0.548 | 100% |
| Ref. 1 | 4.1 | 47.8 | 19.3 | 0.436 | 0.545 | 80% |

As indicated in Table, 1, compared to the OLED in which the first host was not substituted with deuterium in Ref. 1, the OLED in which only the nuclear atoms in phenyl group linked to the triazine as the electron acceptor moiety were substituted with deuterium in Ex. 1 showed equivalent driving voltage, but improved its current efficiency, EQE and lift time by 7.5%, 8.8% and 25%, respectively.

### Experimental Example 2: Measurement of Luminous Properties of OLED

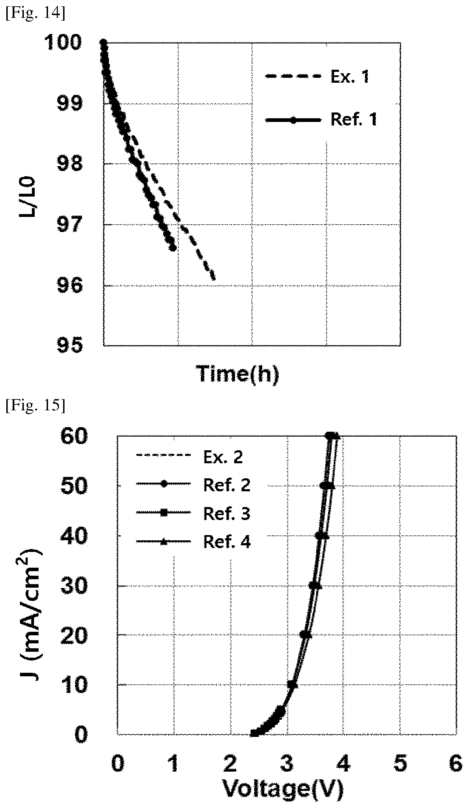

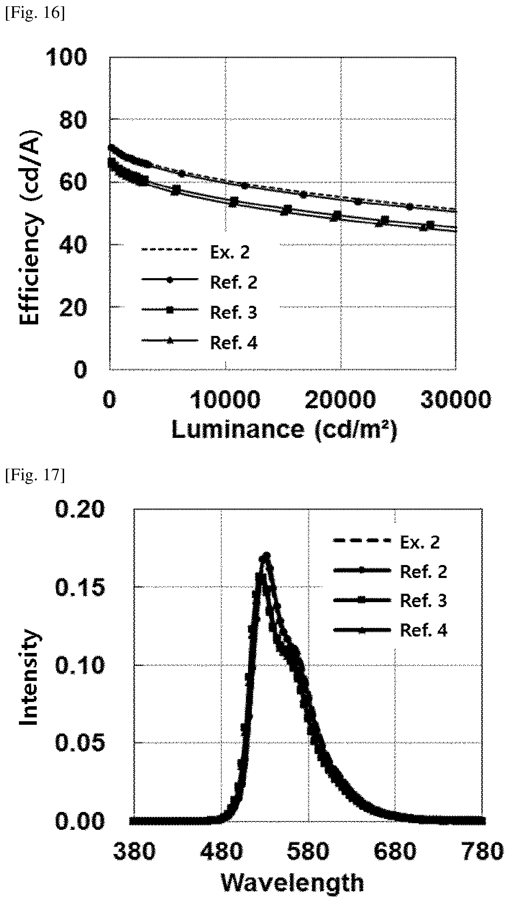

The luminous properties for each of the OLEDs fabricated by Ex. 2 and Ref. 2-4 were measured using the same process as the Experimental Example 1. The measurement results are shown in the following Table 2 and FIGS. 15-18.

**[Table 2]**

| Luminous Properties of OLED | | | | | | LT10 mA/cm ² |
|---|---|---|---|---|---|---|
| I-V-L 10 mA/cm ² | | | | | | |
| Sample | V | cd/A | EQE (%) | CIE(x) | CIE(y) | |
| Ex. 2 | 3.09 | 63.23 | 16.51 | 0.363 | 0.612 | 100% |
| Ref. 2 | 3.08 | 62.53 | 16.24 | 0.357 | 0.618 | 100% |
| Ref. 3 | 3.09 | 57.72 | 15.21 | 0.343 | 0.625 | 50% |
| Ref. 4 | 3.12 | 56.75 | 14.95 | 0.344 | 0.624 | 50% |

As indicated in Table 2, the OLED in which only the nuclear atoms of the phenyl group linked to the triazine were substituted with deuterium in Ex. 1 showed equivalent or somewhat improved luminous efficiency and luminous life time as the OLED in which the whole nuclear atoms in the molecule were substituted with deuterium in Ref. 2.

Also, compared to the OLED in which only the nuclear atoms of the indolocarbazole moiety as the electron donor moiety was substituted with deuterium in Ref. 3, the OLED in Ex. 2 showed equivalent driving voltages, but improved its current efficiency, EQE and lift time by 8.3%, 8.5% and 100%, respectively. Particularly, compared to the OLED in which the first host was not substituted with deuterium in Ref. 4, the OLED in Ex. 2 showed equivalent driving voltage, but improved its current efficiency, EQE and life time by 11.4%, 10.4% and 100%, respectively.

### Experimental Example 2: Measurement of Luminous Properties of OLED

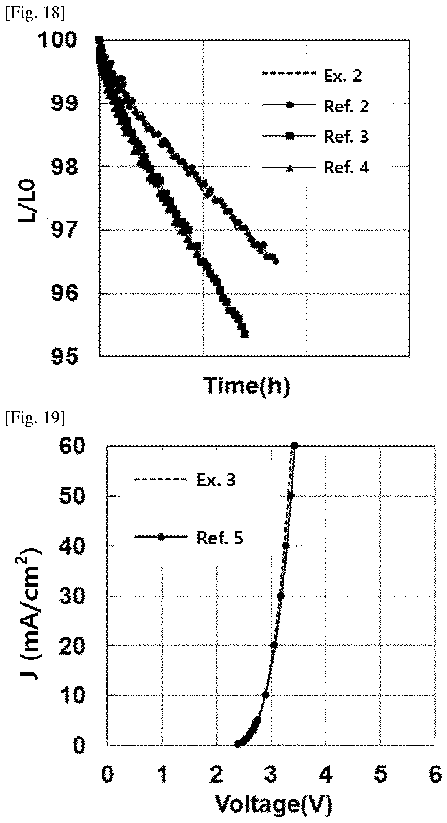

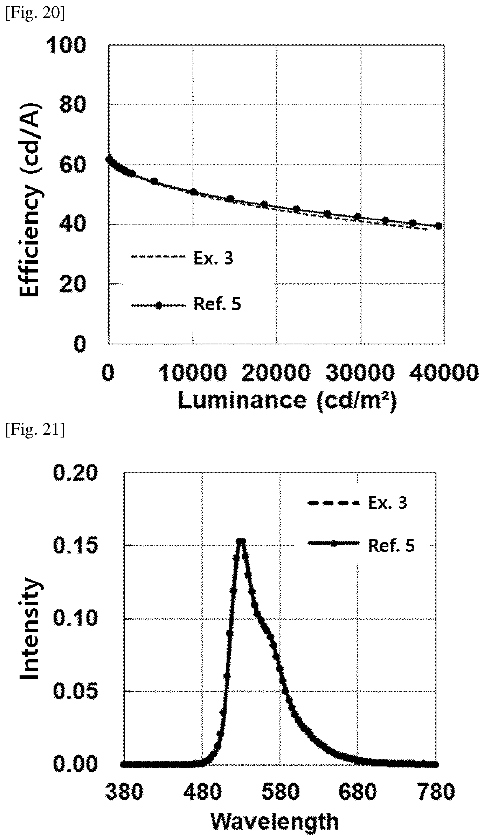

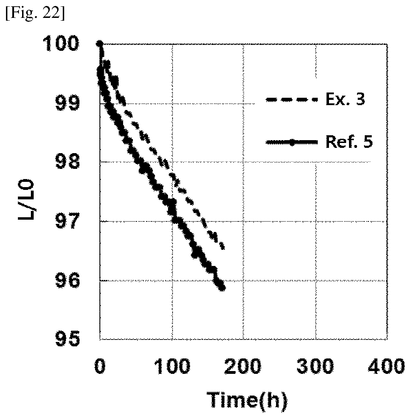

The luminous properties for each of the OLEDs fabricated by Ex. 3 and Ref. 5 were measured using the same process as the Experimental Example 1. The measurement results are shown in the following Table 3 and FIGS. 19-22.

**[Table 3]**

| Luminous Properties of OLED | | | | | | LT mA/cm² |
|---|---|---|---|---|---|---|
| I-V-L 10 mA/cm² | | | | | | |
| Sample | V | cd/A | EQE (%) | CIE(x) | CIE(y) | |
| Ex. 3 | 2.89 | 53.97 | 13.97 | 0.350 | 0.624 | 100% |
| Ref. 5 | 2.89 | 54.24 | 13.97 | 0.350 | 0.624 | 85% |

As indicated in Table 3, compared to the OLED in which the first host was not substituted with deuterium in Ref. 1, the OLED in which the nuclear atoms in phenyl group linked to the triazine as the electron acceptor moiety and the nuclear atoms of the phenylene moiety as the aromatic linker were substituted with deuterium in Ex. 3 showed equivalent driving voltage, current efficiency and EQE, but improved its life time by 17.6%.

Summarizing the results in Experimental Examples 1 to 3, when an organic compound in which only the nuclear atoms constituting a specific moiety is substituted with deuterium is introduced into an OLED, it can be seen that the OLED achieved equivalent luminous efficiency and luminous life time as an OLED in which an organic compound in which all the nuclear atoms of the entire molecule are substituted with deuterium.

The following numbered clauses describe further embodiments:

### [CLAUSE 1]

An organic light emitting diode, comprising:
a first electrode;
a second electrode facing the first electrode; and
at least one emitting unit disposed between the first and second electrodes and including an emitting material layer,
wherein the emitting material layer comprises an organic compound having the following structure of Chemical Formula 1: 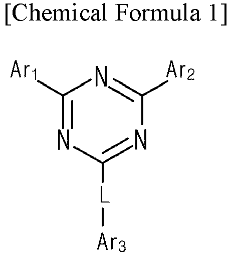
wherein each of Ar₁ and Ar₂ is independently an unsubstituted or substituted C₆~C₃₀ aromatic group or an unsubstituted or substituted C₃~C₃₀ hetero aromatic group; L is an unsubstituted or substituted C₆~C₃₀ arylene group or an unsubstituted or substituted C₃~C₃₀ hetero arylene group; Ar₃ has the following structure of Chemical Formula 2, wherein at least one of nuclear atoms of an aromatic ring and a hetero aromatic ring constituting at least one of Ar₁, Ar₂ and L is substituted with deuterium: 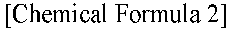 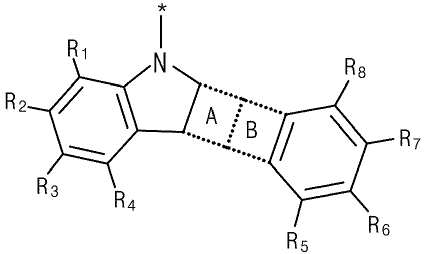
wherein each of R₁ to R₈ is independently selected from the group consisting of hydrogen, an halogen atom, a cyano group, a nitro group, an amino group, a C₁~C₁₀ alkyl halide group, an unsubstituted or substituted linear or branched C₁~C₁₀ alkyl group, an unsubstituted or substituted C₁~C₁₀ alkoxy group, an unsubstituted or substituted C₆~C₃₀ aromatic group and an unsubstituted or substituted C₃~C₃₀ hetero aromatic group; A is a fused aromatic group having the following structure of Chemical Formula 3A; and B is fused hetero aromatic group having the following structure of Chemical Formula 3B: 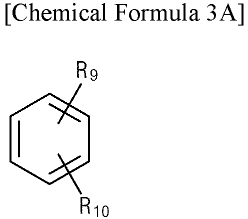
wherein each of R₉ and R₁₀ is independently selected from the group consisting of hydrogen, an halogen atom, a cyano group, a nitro group, an amino group, a C₁~C₁₀ alkyl halide group, an unsubstituted or substituted linear or branched C₁~C₁₀ alkyl group, an unsubstituted or substituted C₁~C₁₀ alkoxy group, an unsubstituted or substituted C₆~C₃₀ aromatic group and an unsubstituted or substituted C₃~C₃₀ hetero aromatic group: 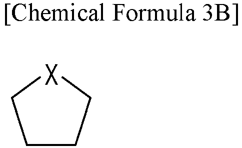
wherein X is oxygen (O), sulfur (S) or NR₁₁, and wherein R₁₁ is selected from the group consisting of protium, deuterium, an halogen atom, a cyano group, a nitro group, an amino group, a C₁~C₁₀ alkyl halide group, an unsubstituted or substituted linear or branched C₁~C₁₀ alkyl group, an unsubstituted or substituted C₁~C₁₀ alkoxy group, an unsubstituted or substituted C₆~C₃₀ aromatic group and an unsubstituted or substituted C₃~C₃₀ hetero aromatic group.

### [CLAUSE 2]

The organic light emitting diode of CLAUSE 1, wherein at least one of nuclear atoms of Ar₁ moiety and Ar₂ moiety is substituted with deuterium.

### [CLAUSE 3]

The organic light emitting diode of CLAUSE 1, wherein at least one of nuclear atoms of L moiety is substituted with deuterium.

### [CLAUSE 4]

The organic light emitting diode of CLAUSE 1, wherein nuclear atoms of Ar₃ moiety is not substituted with deuterium.

### [CLAUSE 5]

The organic light emitting diode of CLAUSE 1, wherein Ar₃ comprises anyone having the following structures of Chemical Formulae 4A to 4F: 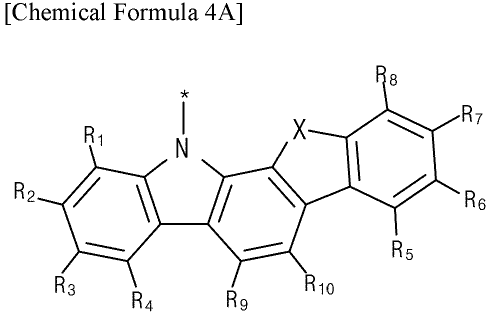 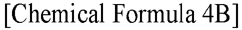 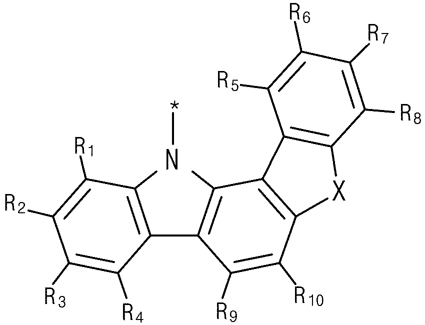 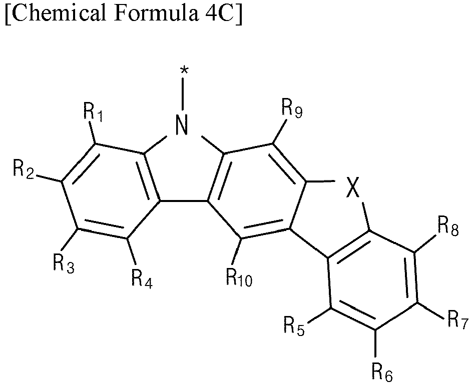 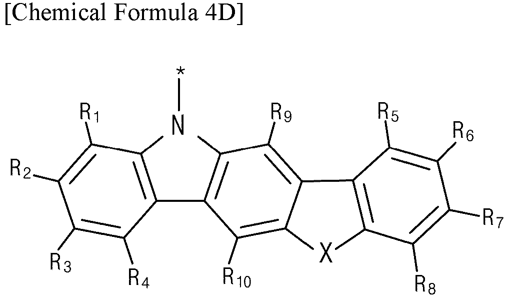 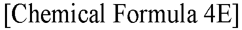 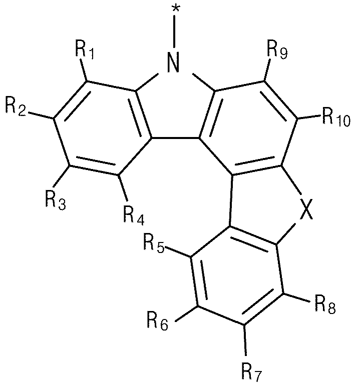 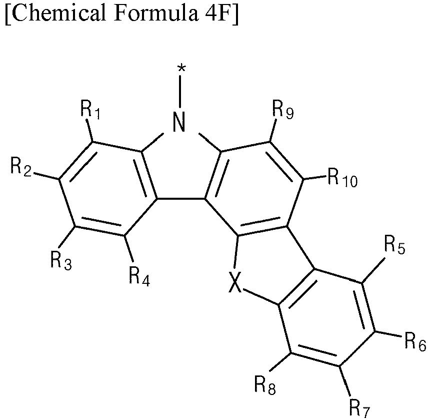 wherein each of R₁ to R₁₀ and X is identical to as defined in Chemical Formulae 2, 3A and 3B.

### [CLAUSE 6]

The organic light emitting diode of CLAUSE 5, wherein Ar₃ comprises anyone having the structure of Chemical Formula 4C or Chemical Formula 4D.

### [CLAUSE 7]

The organic light emitting diode of CLAUSE 1, wherein the organic compound is selected from:

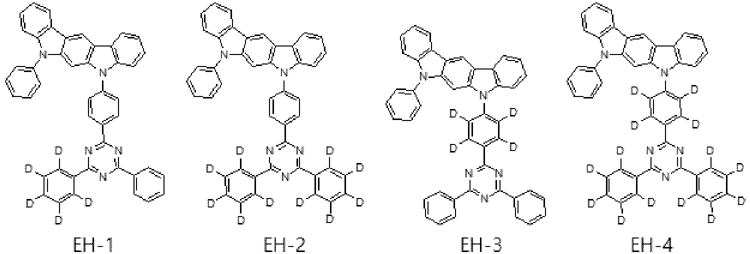

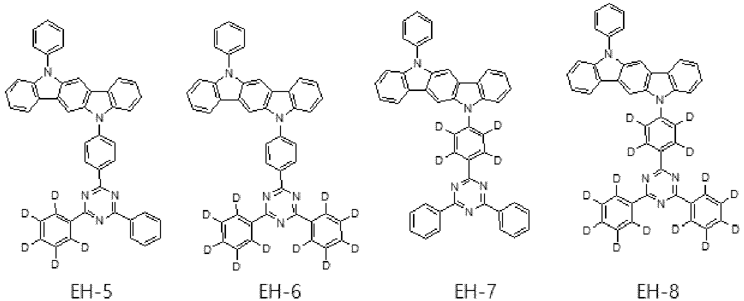

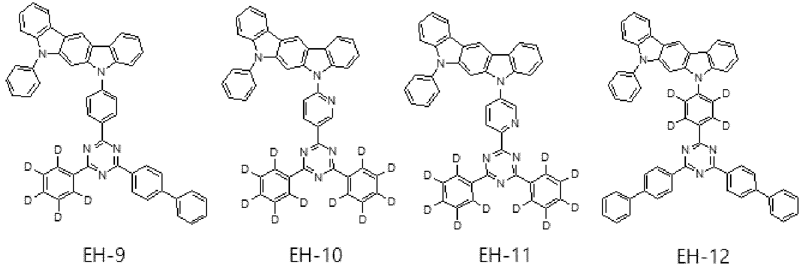

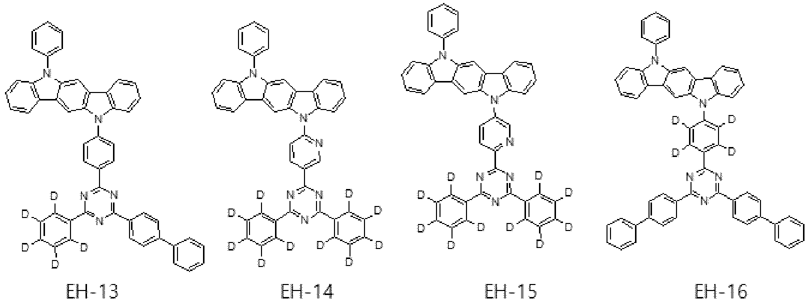

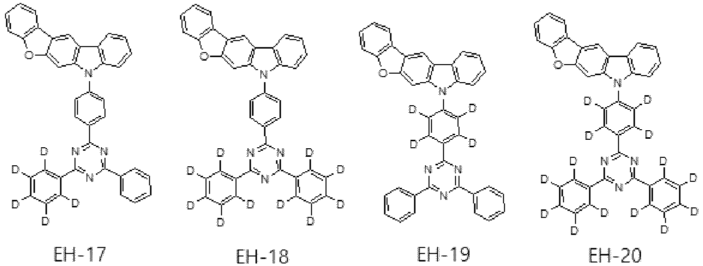

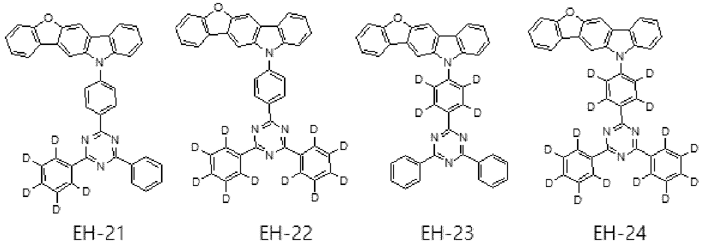

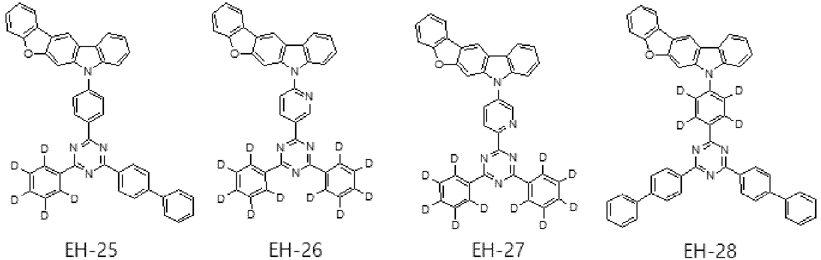

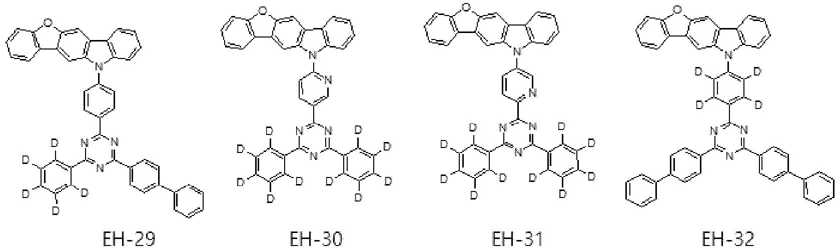

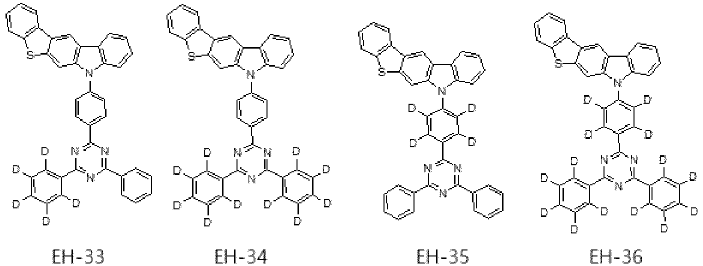

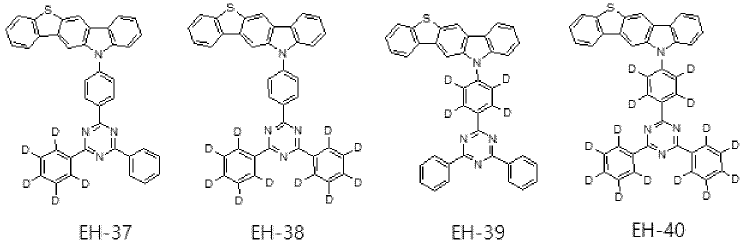

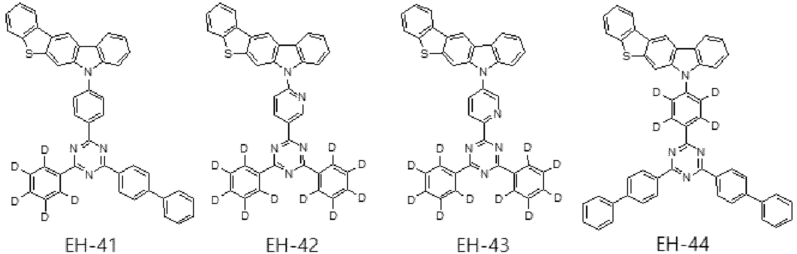

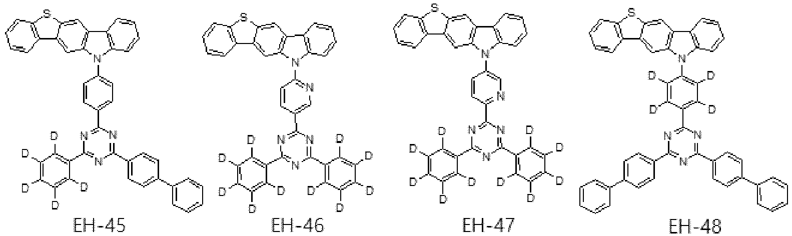

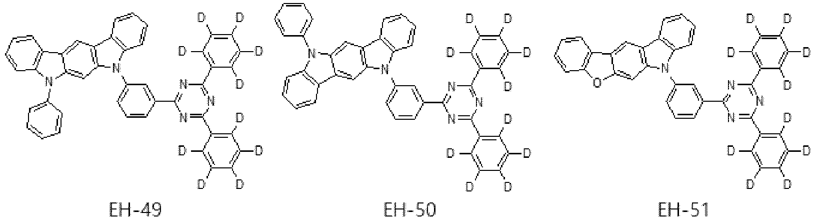

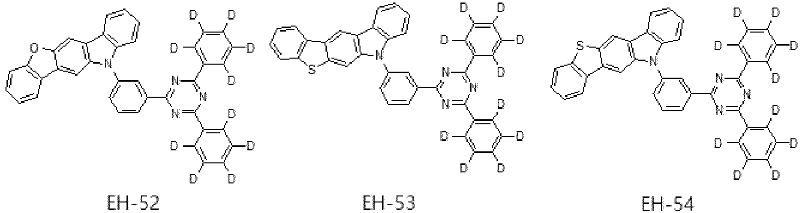

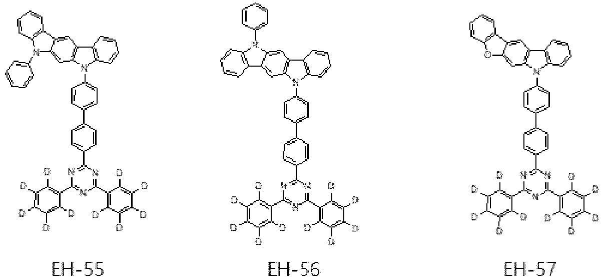

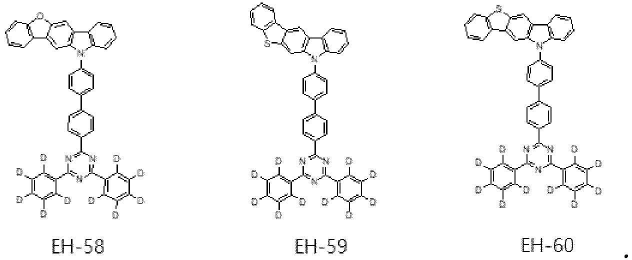

### [CLAUSE 8]

The organic light emitting diode of CLAUSE 1, wherein the emitting material layer comprises a first host and a first dopant,
and wherein the first host comprises the organic compound.

### [CLAUSE 9]

The organic light emitting diode of CLAUSE 8, wherein the first dopant comprises green dopant.

### [CLAUSE 10]

The organic light emitting diode of CLAUSE 8, wherein the emitting material layer further comprises a second host.

### [CLAUSE 11]

The organic light emitting diode of CLAUSE 10, wherein the second host comprises green host.

### [CLAUSE 12]

The organic light emitting diode of CLAUSE 1, wherein the emitting material layer comprises a lower emitting material layer disposed between the first electrode and the second electrode, and an upper emitting material layer disposed between the lower emitting material layer and the second electrode,
wherein one of the lower emitting material layer and the upper emitting material layer comprises a first host and a first second and the other of the lower emitting material layer and the upper emitting material layer comprises a third host and a second dopant, and
wherein the first host comprises the organic compound.

### [CLAUSE 13]

The organic light emitting diode of CLAUSE 12, wherein the third host comprises at least one of yellow host and a red host, and wherein the second dopant comprises at least one of yellow dopant and red dopant.

### [CLAUSE 14]

The organic light emitting diode of CLAUSE 1, wherein L comprises unsubstituted or substituted phenylene.

### [CLAUSE 15]

The organic light emitting diode of CLAUSE 1, wherein the at least one emitting unit comprises a first emitting unit disposed between the first electrode and the second electrode and including a first emitting material layer, and a second emitting unit disposed between the first emitting unit and the second electrode and including a second emitting material layer,
wherein one of the first emitting material layer and the second emitting material layer comprises the organic compound, and
further comprises a first charge generation layer disposed between the first emitting unit and the second emitting unit.

### [CLAUSE 16]

The organic light emitting diode of CLAUSE 15, one of the first emitting material layer and the second emitting material layer comprises a first host and a first dopant, and
wherein the first host comprises the organic compound.

### [CLAUSE 17]

The organic light emitting diode of CLAUSE 16, wherein the first dopant comprises green dopant.

### [CLAUSE 18]

The organic light emitting diode of CLAUSE 18, one of the first emitting material layer and the second emitting material layer further comprises a second host.

### [CLAUSE 19]

The organic light emitting diode of CLAUSE 18, wherein the second host comprises green host.

### [CLAUSE 20]

The organic light emitting diode of CLAUSE 15, wherein the second emitting material layer comprises a lower emitting material layer disposed between the first charge generation layer and the second electrode, and an upper emitting material layer disposed between the lower emitting material layer and the second electrode,
wherein one of the lower emitting material layer and the upper emitting material layer comprises a first host and a first dopant and the other of the lower emitting material layer and the upper emitting material layer comprises a third host and a second dopant, and
wherein the first host comprises the organic compound.

### [CLAUSE 21]

The organic light emitting diode of CLAUSE 20, wherein the third host comprises at least one of yellow host and red host and the second dopant comprises at least one of yellow dopant and red dopant.

### [CLAUSE 22]

The organic light emitting diode of CLAUSE 15, further comprises a third emitting unit disposed between the second emitting unit and the second electrode and comprising a third emitting material layer, and a second charge generation layer disposed between the second emitting unit and the third emitting unit.

### [CLAUSE 23]

The organic light emitting diode of CLAUSE 22, wherein the second emitting material layer comprises the organic compound.

### [CLAUSE 24]

The organic light emitting diode of CLAUSE 23, wherein each of the first emitting material layer and the third emitting material layer emits blue color.

### [CLAUSE 25]

The organic light emitting diode of CLAUSE 23, wherein the second emitting material layer comprises a first host and a first dopant, and
wherein the first host comprises the organic compound.

### [CLAUSE 26]

The organic light emitting diode of CLAUSE 25, wherein the first dopant comprises green dopant.

### [CLAUSE 27]

The organic light emitting diode of CLAUSE 25, the second emitting material layer further comprises a second host of green host.

### [CLAUSE 28]

The organic light emitting diode of CLAUSE 25, wherein the second emitting material layer comprises a lower emitting material layer disposed between the first charge generation layer and the second charge generation layer, and an upper emitting material layer disposed between the lower emitting material layer and the second charge generation layer,
wherein one of the lower emitting material layer and the upper emitting material layer comprises a first host and a first dopant and the other of the lower emitting material layer and the upper emitting material layer comprises a third host and a second dopant, and
wherein the first host comprises the organic compound.

### [CLAUSE 29]

The organic light emitting unit of CLAUSE 28, wherein the third host comprises at least one of yellow host and red host and the second dopant comprises at least one of yellow dopant and red dopant.

### [CLAUSE 30]

An organic light emitting device, comprising:
a substrate; and
an organic light emitting diode of CLAUSE 1 and disposed over the substrate.

### [CLAUSE 31]

The organic light emitting device of CLAUSE 30, wherein the at least one emitting unit of the organic light emitting diode comprises a first emitting unit disposed between the first electrode and the second electrode and including a first emitting material layer, and a second emitting unit disposed between the first emitting unit and the second electrode and including a second emitting material layer,
wherein one of the first emitting material layer and the second emitting material layer comprises the organic compound, and
the organic light emitting diode further comprises a first charge generation layer disposed between the first emitting unit and the second emitting unit.

### [CLAUSE 32]

The organic light emitting device of CLAUSE 31, wherein the substrate defines a red pixel, a green pixel and a blue pixel and the organic light emitting diode is located correspondingly to the red pixel, the green pixel and the blue pixel, and
further comprises a color filter disposed between the substrate and the organic light emitting diode or over the organic light emitting diode correspondingly to the red pixel, the green pixel and the blue pixel.

### [CLAUSE 33]

The organic light emitting device of CLAUSE 31, wherein the substrate defines a red pixel, a green pixel and a blue pixel and the organic light emitting diode is located correspondingly to the red pixel, the green pixel and the blue pixel, and further comprises a color conversion layer disposed between the substrate and the organic light emitting diode or over the organic light emitting diode correspondingly to the red pixel and the green pixel.

It will be apparent to those skilled in the art that various modifications and variations can be made in the present disclosure without departing from the scope of the invention. Thus, it is intended that the present disclosure cover the modifications and variations of the present disclosure provided they come within the scope of the appended claims.

## Claims

1. An organic light emitting diode (700), comprising:
a first electrode (710);
a second electrode (720) facing the first electrode (710); and
an emissive layer (430B) disposed between the first electrode (710) and the second electrode (720),
wherein the emissive layer (430B) includes:
a first emitting material layer (760) disposed between the first electrode (710) and the second electrode (720);
a second emitting material layer (860) disposed between the first emitting material layer (760) and the second electrode (720);
a third emitting material layer (960) disposed between the second emitting material layer (860) and the second electrode (720); and
at least one charge generation layer (790, 890) disposed between the first emitting material layer (760) and the second emitting material layer (860) or between the second emitting material layer (860) and the third emitting material layer (960), and
wherein at least one of the first emitting material layer (760), the second emitting material layer (860) and the third emitting material layer (960) comprises an organic compound having a triazine moiety substituted with at least one of an aromatic group and a hetero aromatic group, and wherein at least one of nuclear atoms among at least one of the aromatic group and the hetero aromatic group is substituted with deuterium.

2. The organic light emitting diode (700) of claim 1, wherein the at least one charge generation layer (790, 890) includes:
a first charge generation layer (790) disposed between the first emitting material layer (760) and the second emitting material layer (860); and
a second charge generation layer (890) disposed between the second emitting material layer (860) and the third emitting material layer (960).

3. The organic light emitting diode of claim 2, wherein the first emitting material layer (760) is included in a first emitting unit (730) disposed between the first electrode (710) and the first charge generation layer (790), the second emitting material layer (860) is included in a second emitting unit (830) disposed between the first charge generation layer (790) and the second charge generation layer (890) and the third emitting material layer (960) is included in a third emitting unit (930) disposed between the second charge generation layer (890) and the second electrode (720).

4. The organic light emitting diode (700A) of claim 2, wherein the second emitting material layer (860A) comprises a lower emitting material layer (862) disposed between the first charge generation layer (790) and the second charge generation layer (890), and an upper emitting material layer (864) disposed between the lower emitting material layer (862) and the second charge generation layer (890),
wherein one of the lower emitting material layer (862) and the upper emitting material layer (864) comprises a first host and a first dopant and the other of the lower emitting material layer (862) and the upper emitting material layer (864) comprises a third host and a second dopant, and
wherein the first host comprises the organic compound,
wherein, preferably, the third host comprises at least one of yellow host and red host and the second dopant comprises at least one of yellow dopant and red dopant.

5. The organic light emitting diode (700) of claim 1, wherein each of the first emitting material layer (760), the second emitting material layer (860) and the third emitting material layer (960) emits independently one of a red color, a green color and a blue color.

6. The organic light emitting diode (700) of claim 5, wherein one of the first emitting material layer (760), the second emitting material layer (860) and the third emitting material layer (960) emits the green color, another of the first emitting material layer (760), the second emitting material layer (860) emits the red color, and the third of the first emitting material layer (760), the second emitting material layer (860) and the third emitting material layer (960) emits the blue color.

7. The organic light emitting diode (700) of claim 6, wherein the second emitting material layer (860) emits the green color.

8. The organic light emitting diode (700) of claim 7, wherein one of the first emitting material layer (760) or the third emitting material layer (960) emits the red color and the other of the first emitting material layer (760) or the third emitting material layer (960) emits the blue color.

9. The organic light emitting diode (700) of claim 1, wherein the second emitting material layer (860) comprises a first host and a first dopant, and
wherein the first host comprises the organic compound.

10. The organic light emitting diode (700) of claim 9, wherein the first dopant comprises green dopants, or wherein the second emitting material layer (860) further comprises a second host of green host.

11. The organic light emitting diode of claim 1, wherein the organic compound has the following structure of Chemical Formula 1: 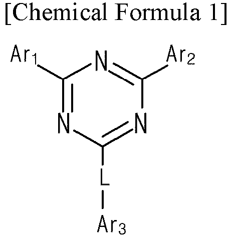
wherein each of Ar₁ and Ar₂ is independently an unsubstituted or substituted C₆~C₃₀ aromatic group or an unsubstituted or substituted C₃~C₃₀ hetero aromatic group; L is an unsubstituted or substituted C₆~C₃₀ arylene group or an unsubstituted or substituted C₃~C₃₀ hetero arylene group; Ar₃ has the following structure of Chemical Formula 2, wherein at least one of nuclear atoms of an aromatic ring and a hetero aromatic ring constituting at least one of Ar₁, Ar₂ and L is substituted with deuterium: 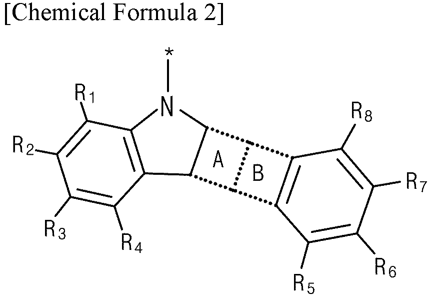
wherein each of R₁ to R₈ is independently selected from the group consisting of hydrogen, an halogen atom, a cyano group, a nitro group, an amino group, a C₁~C₁₀ alkyl halide group, an unsubstituted or substituted linear or branched C₁~C₁₀ alkyl group, an unsubstituted or substituted C₁~C₁₀ alkoxy group, an unsubstituted or substituted C₆~C₃₀ aromatic group and an unsubstituted or substituted C₃~C₃₀ hetero aromatic group; A is a fused aromatic group having the following structure of Chemical Formula 3A; and B is fused hetero aromatic group having the following structure of Chemical Formula 3B: 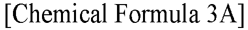 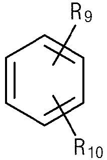
wherein each of R₉ and R₁₀ is independently selected from the group consisting of hydrogen, an halogen atom, a cyano group, a nitro group, an amino group, a C₁~C₁₀ alkyl halide group, an unsubstituted or substituted linear or branched C₁~C₁₀ alkyl group, an unsubstituted or substituted C₁~C₁₀ alkoxy group, an unsubstituted or substituted C₆~C₃₀ aromatic group and an unsubstituted or substituted C₃~C₃₀ hetero aromatic group: 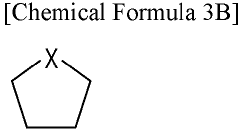
wherein X is oxygen (O), sulfur (S) or NR₁₁, and wherein R₁₁ is selected from the group consisting of protium, deuterium, an halogen atom, a cyano group, a nitro group, an amino group, a C₁~C₁₀ alkyl halide group, an unsubstituted or substituted linear or branched C₁~C₁₀ alkyl group, an unsubstituted or substituted C₁~C₁₀ alkoxy group, an unsubstituted or substituted C₆~C₃₀ aromatic group and an unsubstituted or substituted C₃~C₃₀ hetero aromatic group,
wherein at least one of nuclear atoms of Ar₁ moiety and Ar₂ moiety is substituted with deuterium, and wherein at least one of nuclear atoms of L moiety is substituted with deuterium, and
wherein nuclear atoms of Ar₃ moiety are not substituted with deuterium.

12. The organic light emitting diode (700) of claim 11, wherein Ar₃ comprises anyone having the following structures of Chemical Formulae 4A to 4F: 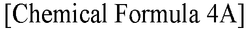 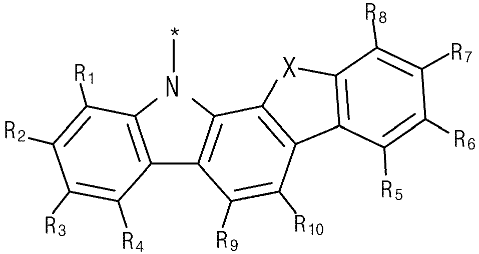 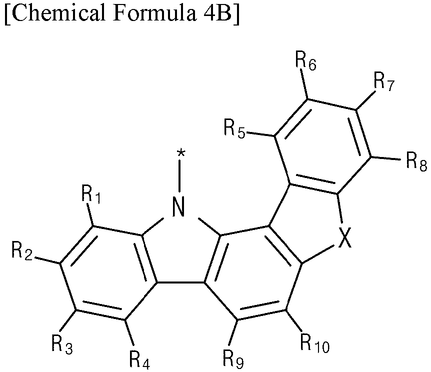 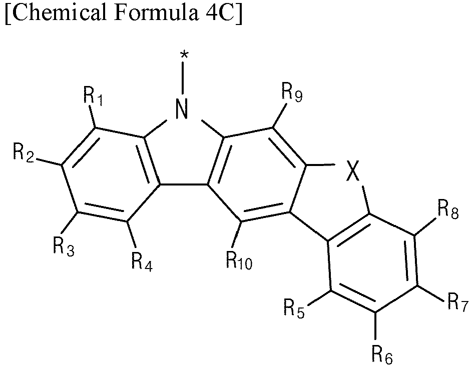 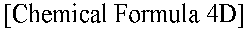 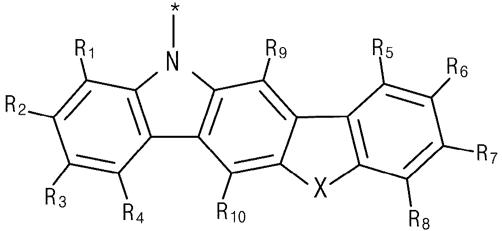 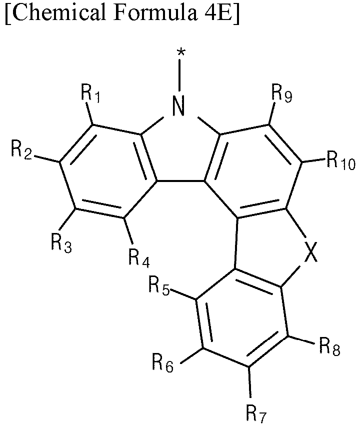 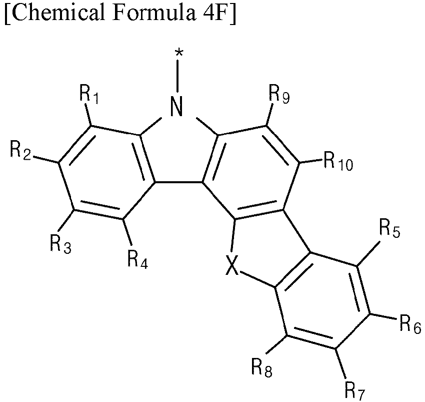
wherein each of R₁ to R₁₀ and X is identical to as defined in Chemical Formulae 2, 3A and 3B,
wherein, preferably, Ar₃ comprises anyone having the structure of Chemical Formula 4C or Chemical Formula 4D.

13. The organic light emitting diode (700) of claim 11, wherein the organic compound is selected from:

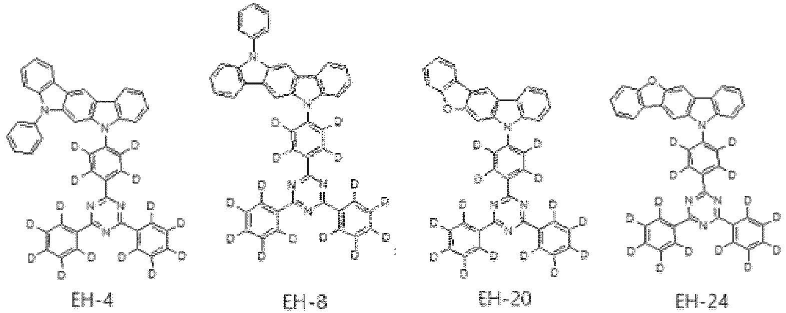

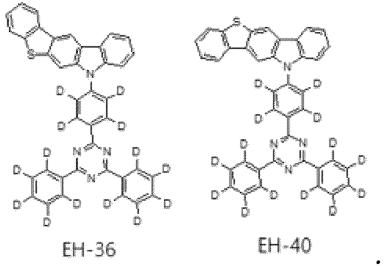

14. The organic light emitting diode (700) of claim 11, wherein L comprises unsubstituted or substituted phenylene.

15. An organic light emitting device (300), comprising:
a substrate (302); and
an organic light emitting diode (700) of claim 1 and disposed over the substrate (302).

16. The organic light emitting device (100, 300) of claim 15, further comprising:
a thin film transistor (Tr) on the substrate (102, 302) and including a semiconductor layer (110, 310), a gate electrode (130, 330), a source electrode (152, 352) and a drain electrode (154, 354); and
an encapsulation film (180) including a first inorganic insulating film (182) on the organic light emitting diode (700), an organic insulating film (184) on the first inorganic insulating film (182) and a second inorganic insulating film (186) on the organic insulating film (184).

17. The organic light emitting device (100, 300) of claim 16, wherein the semiconductor layer (110, 310) includes oxide semiconductor material.

18. The organic light emitting device (100, 300, 1000) of claim 16, further comprising:
a second substrate (304) on the encapsulation film (180);
a color conversion layer (1080) disposed between the organic light emitting diode (700) and the second substrate (304); and
a color filter layer (380) disposed between the color conversion layer (1080) and the second substrate (304).

19. The organic light emitting device (1000) of claim 18, wherein the color conversion layer (1080) includes a quantum dot.
